# EUROPEAN PATENT SPECIFICATION

(11) **EP 4 086 628 B1**
(45) Date of publication and mention of the grant of the patent: **03.07.2024**
(21) Application number: 22172073.3
(22) Date of filing: 06.05.2022
(51) Int. Cl.: G01N 33/52, G01N 33/574

(54) **METHOD, DEVICE, AND KIT FOR POPULATION SCREENING FOR CANCER, CANCER RECURRENCE AND PRECANCEROUS CONDITIONS IN SYMPTOM FREE INDIVIDUALS**
VERFAHREN, VORRICHTUNG UND KIT ZUR UNTERSUCHUNG DER BEVÖLKERUNG AUF KREBS, KREBSREZIDIVE UND KREBSVORSTUFEN BEI SYMPTOMFREIEN PERSONEN
PROCÉDÉ, DISPOSITIF, ET KIT POUR LE DÉPISTAGE DE MASSE DU CANCER, DE LA RÉCIDIVE DU CANCER ET DES ÉTATS PRÉCANCÉREUX CHEZ LES INDIVIDUS SANS SYMPTÔMES

(30) Priority: 07.05.2021 US 202117314502
(43) Date of publication of application: 09.11.2022
(73) Proprietor: Shamsuddin, Abulkalam, Mohammed, Lutherville MD 21093 (US); Vanderlinden, Kim, Coronado, MD 92178 (US)
(72) Inventor: Shamsuddin, Abulkalam, Mohammed, Lutherville MD 21093 (US); Vanderlinden, Kim, Coronado, MD 92178 (US)
(74) Representative: Prüfer & Partner mbB Patentanwälte · Rechtsanwälte

(56) References cited:
- US-A1- 2021 062 241
- US-A1- 2021 263 036
- KREPINSKY J J ET AL: "FROM T-ANTIGEN TO PLASMALOGEN-DERIVED ALDEHYDES: THE IDENTIFICATION OF A MARKER OF COLORECTAL CANCER IN HUMAN RECTAL MUCOUS", CANADIAN JOURNAL OF CHEMISTRY, NRC RESEARCH PRESS, CA, vol. 81, no. 1, 1 January 2003 (2003-01-01), pages 109-117, XP001189022, ISSN: 0008-4042, DOI: 10.1139/V02-195

## Description

### CROSS-REFERENCE TO RELATED APPLICATIONS

### FIELD

This invention relates to an in vitro screening test for precancerous conditions in otherwise healthy populations and/or individuals. Also disclosed herein is a kit containing the components necessary for conducting the test.

### BACKGROUND

Cancer is a major public health problem in the world. Even as pharmaceutical agents for the treatment of cancer are developed, early detection and prevention are still the best hope for combating this human tragedy. In U.S. Pat. No. 4,857,457 and in Shamsuddin et uno., Human Pathology, 19: 7-10, 1988, there is reported a test for colorectal cancer which can detect increased risk of cancer of the large intestine employing rectal mucus. The mucus is reacted with the enzyme galactose oxidase by moistening a cellulose membrane filter, which had previously been impregnated with a phosphate buffer solution of the enzyme and then lyophilized, and then contacting the moistened cellulose membrane filter with a Metricel membrane filter bearing the mucus sample for 1-2 hours. The mucus-bearing membrane filter is then washed with distilled water for 1 minute, reacted with basic fuchsin for 15 minutes, washed in tap water for 10 minutes and then air dried.

This basic procedure, although simple, is currently used for detecting colorectal cancers and precancerous conditions. However, it has since been found that the disclosed screening procedure i.e., using the marker disaccharide employed therein as predictive of colorectal cancer is not restricted to cancers of the large intestine. But rather, for example as described in U.S. Patent Nos. 5,162,202 and 5,348,860 to Shamsuddin, the disaccharide marker may be present in other sites around the body experiencing similar precancerous or cancerous traits. Also, the test as described therein is lengthy.

Krepinsky et al. ("From T-antigen to plasmalogen-derived aldehydes: The identification of a marker of colorectal cancer in human rectal mucous", Canadian Journal of Chemistry, vol. 81, no. 1, 1 January 2003, pp. 109-117) refers to the colour formation in a test for colorectal cancer. It was found that the mucous from colorectal cancer patients contains compounds that form purple adducts with the Schiff reagent directly, and that they therefore do not require oxidation by galactose oxidase.

In U.S. Patent Application No. 16/936,998 to Shamsuddin, a screening test method, device, and kit for mucosal carbohydrates and associated conditions is disclosed. The method tests abnormal carbohydrates in mucus or body fluid using reagents of galactosidase oxidase, and Schiff's Reagent.

Most cancers in humans are believed to be the result of exposure to one or more environmental carcinogens via air we inhale, the food we ingest, water and drinks we consume etc. The carcinogens are excreted through the large intestine or kidneys and urinary bladder, or the lungs, skin (through sweat) etc. Thus, it can be expected that the carcinogen(s) and/or their metabolite(s) cause changes in those organs, in addition to causing corresponding changes simultaneously in the organ bearing the cancer and precancer. Precancer or precancerous conditions are those stages or diseases that render an otherwise healthy, asymptomatic individual highly susceptible to subsequent cancer (high risk symptomatic) as they may progress to cancer. Thus, the presence of, or threat of a cancerous or precancerous condition in the body of an individual, including but not restricted to the large intestine, can be detected accurately by the method of this invention initially using rectal mucus sampling, followed, if necessary, by sampling of other body fluids, such as secretions of breast, prostate, semen, cervical and vaginal mucus, sputum, bronchial or alveolar secretions, until the precise situs of the cancerous or precancerous condition is located, without the prior risk of false negatives which limited the value of this technique as a screening test, particularly as a field test, for the general population. The invention disclosed herein has the unexpected benefit of accurately and reliably detecting cancer and precancerous conditions in any organ in an individual showing no signs or symptoms of a cancerous or precancerous condition.

The disclosure as provided herein can additionally serve to save lives, by providing a routine screening process for people having no obvious signs or symptoms for a precancerous condition. As the old adage says: "an ounce of prevention is better than a pound of cure", not only the disclosure would reduce mortality and morbidity but also in terms of money, it will markedly reduce national healthcare costs for patients and providers alike, globally.

### OBJECTS OF THE INVENTION

It is a general object of the disclosure to provide such a test which can be performed on the public, including those who do not currently present symptoms or otherwise display a need for diagnostic testing, as part of a general population screening practice for example. The screening test as provided herein allow for rapid testing and results thereby facilitating its use in mass testing programs which do not require a special diagnostic laboratory and permitting reporting the results of the test to the individual or individuals tested (or their physician) and/or obtain an additional rectal mucus sample if a negative result is determined to be a false negative, before the individual leaves the testing area.

Yet another object is to provide such a kit.

Still another object of the invention is to provide a kit for conducting the screening test of this invention whose critical components are stable for a protracted period.

Still another object is to provide a method for performing the screening test employing the kit of this invention which can be completed fast enough at the point-of-care or in the subject's home to provide results for the tests to the individuals being tested while they wait.

Upon further study of the specification and appended claims, further objects and advantages of this invention will become apparent to those skilled in the art.

### SUMMARY

This disclosure relates to an in vitro screening test method. Also described herein is a device, and kit for cancerous and precancerous conditions, which are not claimed. Specifically, the method tests abnormal carbohydrates in mucus or body fluid using reagents of galactose oxidase, and Schiff's Reagent. The in vitro screening test method, device, and kit provides improved accuracy, and minimizes handling procedures. This disclosure further relates to the use of the device or kit in a healthcare facility for an initial evaluation for cancerous and precancerous conditions.

In one aspect, the in vitro screening method for cancerous and precancerous conditions and lesions contains the steps of applying mucus or body fluid to a test strip or membrane with galactose oxidase pre-embedded, oxidizing marker carbohydrates in the mucus or body fluid that contains marker carbohydrates by reacting with galactose oxidase, and activating a container with Schiff reagent solution adjacent to the test strip or membrane to contact the test strip or membrane, and wherein the Schiff reagent solution is not initially in contact with the test strip or membrane.

In some embodiments, the in vitro screening method for cancerous and precancerous conditions and lesions contains one or more additional steps selected from: applying water onto the test strip or membrane before applying mucus or body fluid to the test strip or membrane, removing the mucus or body fluid by washing before activating a container with Schiff reagent solution, washing the test strip or membrane with tap water after letting the Schiff reagent solution to contact the test strip or membrane, drying the test strip or membrane, and/or evaluating color of the test strip or membrane.

In another aspect, the device for cancerous and precancerous conditions and lesions contains a test strip or membrane with galactose oxidase pre-embedded and a container with Schiff reagent solution, wherein the Schiff reagent solution is not initially in contact with the test strip or membrane, and can be activated to contact the test strip or membrane after the marker carbohydrate is oxidized by galactose oxidase. The mechanism to activate the carbohydrate Schiff reagent solution (to contact the test strip or membrane after the marker carbohydrate is oxidized by galactose oxidase) is not limited. In one aspect, the mechanism can be a twist valve, a breakable barrier, or the like, wherein after opening the twist valve or breaking the barrier, the Schiff reagent solution contacts the test strip or membrane.

In another aspect, this disclosure describes a testing kit for cancerous and precancerous conditions containing a test strip or membrane with galactose oxidase pre-embedded and a container with Schiff reagent solution. In one aspect, the test strip or membrane with galactose oxidase pre-embedded further contains dry culture medium, wherein the dry culture medium can activate the galactose oxidase once water is added onto the test strip or membrane. In another aspect, the pre-embedded galactose oxidase in the test strip or membrane is microencapsulated.

In one aspect, the in vitro screening method for cancerous and precancerous conditions and lesions contains the steps of applying mucus or body fluid to a test strip or membrane with a Schiff reagent pre-embedded, oxidizing marker carbohydrates in the mucus or body fluid that contains marker carbohydrates by reacting with galactose oxidase, and activating the Schiff reagent embedded in the test strip or membrane to contact the test strip or membrane.

In some embodiments, the in vitro screening method for cancerous and precancerous conditions and lesions contains one or more additional steps selected from: applying water onto the test strip or membrane before applying mucus or body fluid to the test strip or membrane, removing the mucus or body fluid by washing before activating the Schiff reagent, washing the test strip or membrane with tap water after letting the Schiff reagent contact the sample, drying the test strip or membrane, and/or evaluating color of the test strip or membrane.

In another aspect, the device for cancerous and precancerous conditions and lesions contains a test strip or membrane with a Schiff reagent pre-embedded and a container with a galactose oxidase solution, wherein the Schiff reagent can be activated to contact the sample after the marker carbohydrate is oxidized by galactose oxidase. The mechanism to activate the Schiff reagent solution (to contact the sample after the marker carbohydrate is oxidized by galactose oxidase) is not limited. In one aspect, the mechanism can be by addition of a pore-forming agent.

In another aspect, this disclosure describes a testing kit for cancerous and precancerous conditions containing a test strip or membrane with a Schiff reagent pre-embedded and a container with galactose oxidase reagent solution. In one aspect, the test strip or membrane with Schiff reagent pre-embedded further contains dry culture medium, wherein the dry culture medium can activate the galactose oxidase once water is added onto the test strip or membrane. In another aspect, the Schiff reagent in the test strip or membrane is microencapsulated.

In another aspect, the invention provides an in vitro screening method for rapidly testing a subject for precancerous condition, where the subject has no obvious signs or symptoms of a cancerous or precancerous condition, is disclosed. The method comprises, at step (a), assaying a portion of a biological sample obtained from an individual for the presence therein of glycoprotein containing at least one carbohydrate selected from the group consisting of: beta-D-Gal-(1->3)-D-GalNAc, Fuc-alpha-1->2 Gal-Beta(1->4)-Fuc-alpha-1->3-GlcNAc, Fuc-Alpha-1->2 Gal-beta-(1->4)-Fuc-alpha-1->3-GlcNAc-beta-(1->3)-Gal-beta-(1>4)-GlcNAc and Fuc-alpha-1->2-Gal-beta-(1->4)-Fuc-alpha-1->3-GlcNAc-beta-(1->3)-Gal-beta-(1->4)-Fuc-alpha-1->3-GlcNAc. Assaying the biological sample can include briefly subjecting the sample to oxidizing conditions which are capable of selectively oxidizing only the cyclic sugar moieties of any said marker carbohydrate present in the glycoprotein in the sample at a hydroxy group-bearing ring carbon atom thereof to form an aldehydic sugar moiety, and then visualizing any aldehydic saccharide groups thus formed with Schiff's base decolorized dye. Next, at step (b), a portion of the biological sample can also be assayed for the presence therein of any glycoprotein by subjecting the sample to the oxidizing action of an oxidizing agent which oxidizes the saccharide moieties of any glycoprotein therein to aldehydic sugar moieties, and then visualizing any thus-produced aldehydic sugar moieties. The presence of the formed aldehydic sugar moieties can confirm the adequacy of the sampling and the absence of the formed aldehyde sugar moieties can establish that the negative test results were due to sampling error. At step (c), the method can include retesting the individual in the same manner as provided above with a fresh biological sample, if the first sample assays negative in steps (a) and (b). Finally, at step (d), the method can include diagnosing the asymptomatic patient as having cancer or a precancerous condition based on visualizing a color change in the Schiff's base dye.

The assay can comprise the steps of adsorbing the biological sample onto a protein-capturing water-insoluble substrate, and then washing the substrate to remove non-immobilized components of the sample from the substrate. The insoluble substrate can be a membrane filter.

The marker carbohydrate sugar moieties can be assayed by selectively oxidizing the glycoprotein so as to selectively oxidize the primary hydroxy groups of any galactose moieties thereof to aldehydic groups and the thus-oxidized glycoprotein is then assayed for oxidized vicinal galactose moieties. The galactose moieties can be oxidized with galactose oxidase, and the oxidized vicinal galactose moieties can be visualized with basic fuchsin. Any aldehydic sugar moieties in a sample can be further oxidized with periodic acid and visualized with basic fuchsin.

The method as described above can be performed simultaneously on a plurality of biological samples obtained from a plurality of individuals as part of a field screening for cancer.

Step (b) can be conducted concurrently with step (a) on a different portion of the same biological sample such that immediately thereafter, if a negative result is obtained in both of steps (a) and (b), another biological sample can be collected from the individual, and steps (a) and (b) can be repeated.

Prior to assaying, the biological sample can be adsorbed onto a protein-capturing water-insoluble substrate, and the substrate washed to remove the non-immobilized components of the biological sample from the substrate. The sample is then assayed in step (a) by selectively oxidizing any glycoprotein in the sample so as to oxidize the primary hydroxy group of the galactose sugar moiety of any said carbohydrate therein to an aldehydic group and the thus-oxidized galactose moiety can then be visualized with basic fuchsin. A sample which tests negative in that assay can be further oxidized with periodic acid and the aldehydic sugar moiety in the thus-oxidized sample is visualized with basic fuchsin.

The insoluble substrate can be a membrane filter and the galactose sugar moieties can be oxidized with galactose oxidase. The oxidizing agent for the galactose moieties can be present in the insoluble substrate or can be applied directly thereto after the sample is applied thereto.

In embodiments, the insoluble substrate can be a membrane filter, and the galactose sugar moieties can be oxidized with galactose oxidase, where the galactose oxidase is applied directly to the membrane filter after the biological sample is applied thereto.

An in vitro method for screening for a precancerous condition in an organ other than the large intestine of a human being is disclosed, where the human being has no obvious signs or symptoms for a cancerous or precancerous condition. The method can include subjecting a sample of proteinaceous secretion or proteinaceous fluid other than rectal mucus associated with that organ, to an assay for the presence therein of a marker carbohydrate selected from the group consisting of beta-D-Gal-(1->3)-D-GalNAc, Fuc-alpha-1->2 Gal-Beta(1->4)-Fuc-alpha-1->3-GlcNAc, Fuc-Alpha-1->2 Gal-beta-(1->4)-Fuc-alpha-1->3-GlcNAc-beta-(1->3)-Gal-beta-(1>4)-GlcNAc and Fuc-alpha-1->2-Gal-beta-(1->4)-Fuc-alpha-1->3-GlcNAc-beta-(1->3)-Gal-beta-(1->4)-Fuc-alpha-1->3-GlcNAc.

Prior to assaying, the proteinaceous secretion can be adsorbed onto a protein-capturing water-insoluble substrate, and the substrate then washed to remove the nonimmobilized components of the sample from the substrate. The sample can be assayed for the marker carbohydrates by selectively oxidizing any glycoprotein in the sample with galactose oxidase so as to selectively oxidize the primary hydroxy group of any galactose sugar moiety of any said marker carbohydrate therein to an aldehydic group. The thus-oxidized galactose moiety can then be visualized with basic fuchsin. A portion of the sample which tests negative in that assay can be further oxidized with periodic acid, and the aldehydic sugar moiety in the thus-oxidized sample can be visualized with basic fuchsin.

The portion of the sample of proteinaceous secretion which is oxidized with periodic acid can be a different portion of the same sample which is oxidized with galactose oxidase, and can be oxidized concurrently therewith.

The human being can be female, and the biological sample can be any or all of, a vaginal mucus, endocervical mucus, nipple aspirate, sputum, bile duct fluid, rectal mucus, and/or pancreatic duct fluid aspirate. The human being can be male, and the biological sample can be any or all of a prostatic secretion, semen, sputum, bile duct fluid, rectal mucus, and/or, pancreatic duct fluid aspirate.

### BRIEF DESCRIPTION OF THE DRAWINGS

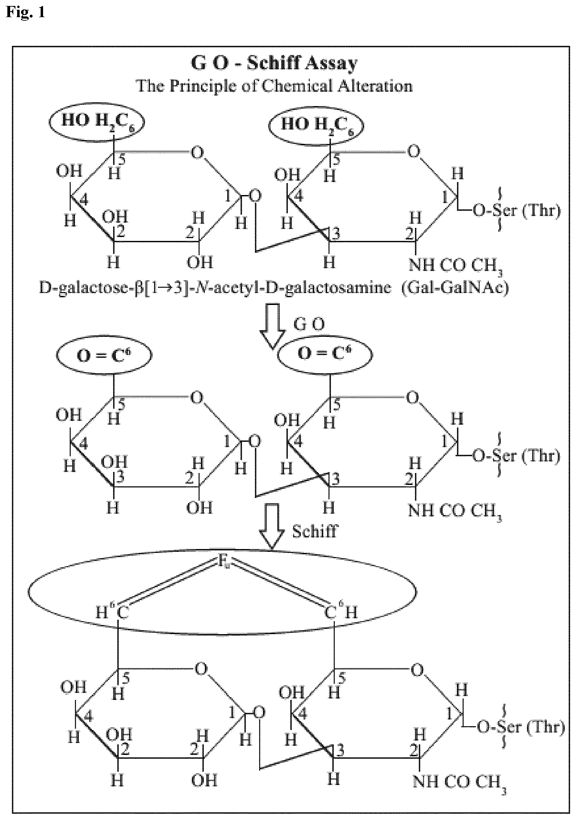

Fig. 1 depicts the principle of the reactions involved in the kits and in vitro methods of the invention. In the figure, the marker Gal-GalNAc is reacted with galactose oxidase (GO) to yield two vicinyl aldehydes at C-6 positions, which then bind to basic fuchsin to impart a magenta color.

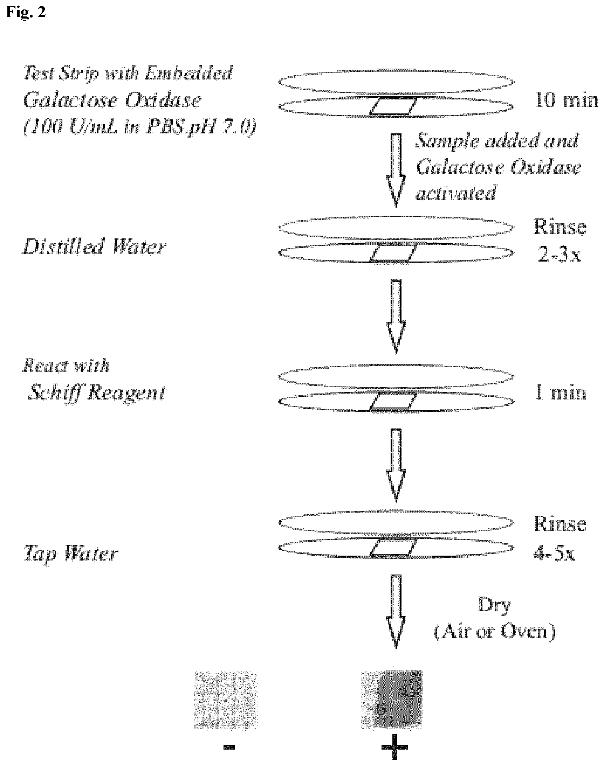

Fig 2. depicts an embodiment of the in vitro methods of the claimed invention wherein samples are reacted on a slide to identify a cancerous or precancerous condition. In samples from subjects not having any cancer or precancer the test panel is colorless, while typically a magenta (with a range of pink to purple) color is indicative of the marker disaccharide specific for cancer and precancerous conditions and lesions.

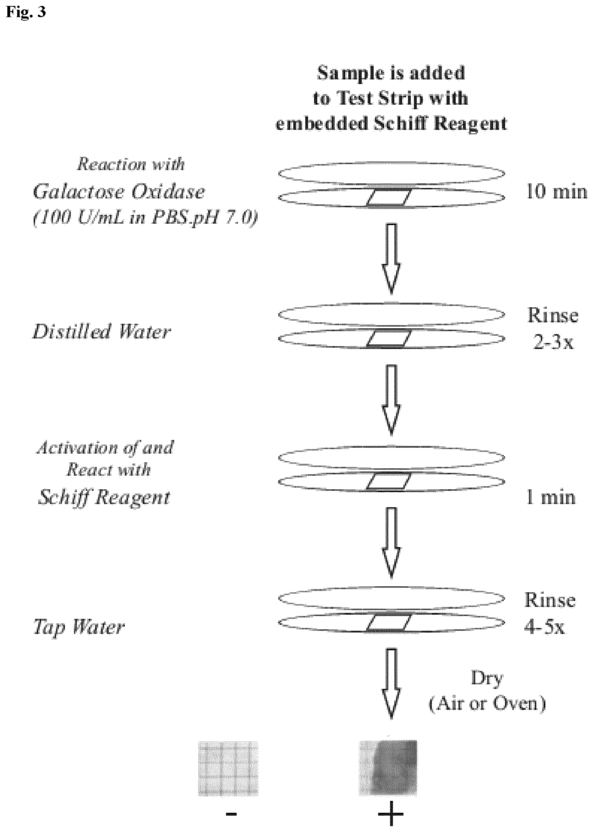

Fig 3. depicts an embodiment of the in vitro methods of the claimed invention where in samples are reacted on a slide to identify a cancerous or precancerous condition. In samples from subjects not having any cancer or precancer the test panel is colorless, while typically a magenta (with a range of pink to purple) color is indicative of the marker disaccharide specific for cancer and precancerous conditions and lesions.

### DETAILED DESCRIPTION

The following is a detailed description provided to aid those skilled in the art in practicing the present disclosure.

Unless otherwise defined, all technical and scientific terms used herein have the same meaning as commonly understood by one of ordinary skill in the art to which this disclosure belongs. The terminology used in the description is for describing particular embodiments only and is not intended to be limiting of the disclosure.

Where a range of values is provided, it is understood that each intervening value, to the tenth of the unit of the lower limit unless the context clearly dictates otherwise (such as in the case of a group containing a number of carbon atoms in which case each carbon atom number falling within the range is provided), between the upper and lower limit of that range and any other stated or intervening value in that stated range is encompassed within the disclosure. The upper and lower limits of these smaller ranges may independently be included in the smaller ranges is also encompassed within the disclosure, subject to any specifically excluded limit in the stated range. Where the stated range includes one or both of the limits, ranges excluding either of those included limits are also included in the disclosure.

All numerical values within the detailed description and the claims herein are modified by "about" or "approximately" the indicated value, and take into account experimental error and variations that would be expected by a person having ordinary skill in the art.

The following terms are used to describe the present disclosure. Unless otherwise defined, all technical and scientific terms used herein have the same meaning as commonly understood by one of ordinary skill in the art to which this disclosure belongs. The terminology used in the description is for describing particular embodiments only and is not intended to be limiting of the disclosure.

The articles "a" and "an" as used herein and in the appended claims are used herein to refer to one or to more than one (i.e., to at least one) of the grammatical object of the article unless the context clearly indicates otherwise. By way of example, "an element" means one element or more than one element.

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of or "exactly one of," or, when used in the claims, "consisting of," will refer to the inclusion of exactly one element of a number or list of elements. In general, the term "or" as used herein shall only be interpreted as indicating exclusive alternatives (i.e., "one or the other but not both") when preceded by terms of exclusivity, such as "either," "one of," "only one of," or "exactly one of."

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of and "consisting essentially of shall be closed or semi-closed transitional phrases, respectively, as set forth in the United States Patent Office Manual of Patent Examining Procedures, Section 2111.03.

As used herein in the specification and in the claims, the phrase "at least one," in reference to a list of one or more elements, should be understood to mean at least one element selected from anyone or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

As used herein in the specification and in the claims, the phrase "marker carbohydrate" or "marker saccharide" should be understood to mean a carbohydrate that can provide or cancerous and precancerous information through the use of the method described herein. The marker carbohydrate includes, but not limited to, beta-D-Gal-(1->3)-D-GalNAc, Fuc-alpha-1->2 Gal-Beta(1->4)-Fuc-alpha-1->3-GlcNAc, Fuc-Alpha-1->2 Gal-beta-(1->4)-Fuc-alpha-1->3-GlcNAc-beta-(1->3)-Gal-beta-(1>4)-GlcNAc and Fuc-alpha-1->2-Gal-beta-(1->4)-Fuc-alpha-1->3-GlcNAc-beta-(1->3)-Gal-beta-(1->4)-Fuc-alpha-1->3-GlcNAc.

As used herein in the specification and in the claims, the phrase "mucus" or "body fluid" should be interchangeable. The phrase "mucus" or "body fluid" should be broadly construed to any fluid or mucus from a human body that contains the marker carbohydrate(s).

As used herein in the specification and in the claims, the phrases "embedded," "impregnated" and "pre-embedded" should be interchangeable. In certain embodiments, the reagents of the invention may be embedded into a membrane or test strip directly by binding or immobilizing the reagent on the surface of the membrane or test strip or by loading the reagent in the pores of a porous membrane, *via* a coating. In still other embodiments the reagents of the invention are encapsulated in a capsule or microcapsule and subsequently embedded, coated, or impregnated into the reagent or test strip. In still other embodiments, the reagents can be utilized as the eluent in a lateral flow assay or lateral flow test strip. In particular embodiments, a fluid containing a Schiff reagent can be used as the eluent in a lateral flow assay or lateral flow test strip. In another particular embodiment, a fluid containing galactose oxidase can be used as the eluent in a lateral flow assay or lateral flow test strip.

The background and theory employed in this disclosure for detecting the presence of the marker carbohydrates in the mucus or body fluid of individuals tested for cancerous or precancerous conditions are disclosed in U.S. Pat. Nos. 4,857,457, 5,162,202, and 5,348,860, as well as in Usefulness of Galactose Oxidase-Schiff Test in Rectal Mucus for Screening of Colorectal Malignancy ANTICANCER RESEARCH 21:1247-1256 (2001). The reaction used in the methods and kits is depicted in Fig. 1.

The present invention is an improvement in the assay as described previously, with respect to minimize the false positives as a result of sampling or procedural error as well as for convenience.

It should also be understood that, in certain methods described herein that include more than one step or act, the order of the steps or acts of the method is not necessarily limited to the order in which the steps or acts of the method are recited unless the context indicates otherwise.

The term "patient" or "subject" is used throughout the specification to describe an animal, preferably a human or a domesticated animal, to whom treatment, including prophylactic treatment, with the compositions according to the present disclosure is provided. For treatment of those infections, conditions or disease states which are specific for a specific animal such as a human patient, the term patient refers to that specific animal, including a domesticated animal such as a dog or cat or a farm animal such as a horse, cow, sheep, etc. In general, in the present disclosure, the term patient refers to a human patient unless otherwise stated or implied from the context of the use of the term.

The "having no obvious signs or symptoms" or "not expressing symptoms" includes otherwise healthy subjects expressing minimal or no symptoms typically associated with cancer or precancer. In certain embodiments, the term "having no obvious signs or symptoms" includes otherwise healthy individuals who are genetically predisposed to certain cancers or who have a family history for such cancer, (e.g., is in a "highly susceptible to subsequent cancer" category). In certain embodiments, the term "having no obvious signs or symptoms" includes otherwise healthy individuals who are at increased risk to certain cancers based on lifestyle, environmental, or other social determinants (e.g., smokers; individuals who work with radiation or hazardous materials; individuals exposed to carcinogens through groundwater; individuals taking medications which increase risk of cancer). In certain embodiments, the term "having no obvious signs or symptoms" includes otherwise healthy individuals who have previously been treated for a cancerous condition which was removed or which entered remission. In such embodiments, the methods of the invention can be used to detect recurrence of a cancerous condition. In certain embodiments, the term having no obvious signs or symptoms includes individuals experiencing symptoms which are not exclusively attributed to cancer or precancer. Such symptoms include, but are not limited to, fatigue, loss of appetite, weight loss, cough, shortness of breath, swollen lymph nodes, indigestion, nausea, and pain. In particular embodiments, a person is considered having no obvious signs or symptoms for lung cancer if they are expressing minimal or no symptoms of pain in the rib or chest area, a chronic or prolonged cough, shortness of breath, and/or wheezing. In particular embodiments, a person is considered having no obvious signs or symptoms for breast cancer if they are expressing minimal or no signs or symptoms of a lump in the breast, breast tenderness, nipple discharge, and/or changes in the shape of the breast or nipple. In particular embodiments, a person is considered having no obvious signs or symptoms for colon cancer if they are expressing minimal or no symptoms of changes in bowel habits, changes in stool consistency, blood in the stool, and/or persistent abdominal discomfort. In particular embodiments, a person is considered having no obvious signs or symptoms for prostate cancer if they are expressing minimal or no symptoms of frequent urination, difficulty starting or maintaining a steady stream of urine, dribbling of urine, excessive urination or urge to urinate, urinary retention, leaking, and/or bone pain. In particular embodiments, a person is considered having no obvious signs or symptoms for endometrial cancer if they are expressing minimal or no symptoms of pain in the pelvis, discomfort or pain during sexual intercourse, abnormal menstruation or spotting, abnormal vaginal bleeding and/or abnormal vaginal discharge. In particular embodiments, a person is considered having no obvious signs or symptoms for ovarian cancer if they are expressing minimal or no symptoms of abdominal or pelvic pain, change in bowel habits, abdominal fullness, fluid in the abdomen, and/or a lump in the abdomen. In particular embodiments, a person is considered having no obvious signs or symptoms for pancreatic cancer if they are expressing minimal or no symptoms of abdominal pain or pain in the middle back, fluid in the abdomen, abnormally dark colored urine, and/or a yellowing of the skin or eyes. In particular embodiment a subject is considered not without obvious signs or symptoms for head & neck cancer is they are not expressing swelling or a sore that does not heal; red or white patch in the mouth; lump, bump, or mass in the head or neck area, with or without pain; persistent sore throat; foul mouth odor not explained by hygiene; hoarseness or change in voice; nasal obstruction or persistent nasal congestion.

Specific embodiments of the technique employed in this invention for detecting the presence of marker carbohydrates in the rectal mucus of symptomatic individuals (showing signs and symptoms of colon and intestinal cancers) are disclosed in U.S. Pat. Nos. 4,857,457 and 5,348,860.

This invention provides a reliable screening tool for the detection of a wide variety of cancers, e.g., lungs, rectum, colon, stomach, kidney, gallbladder, liver, pancreas, prostate, testes, breast, head and neck, cervix, uterus and ovaries. The invention also provides a reliable screening tool for the detection of precancerous conditions or increased risk, for example, if the individual is high risk symptomatic (e.g., as in a "highly susceptible to subsequent cancer" category). For example, a "highly susceptible to subsequent cancer" patient may include individuals who are not expressing signs and symptoms of cancer or precancer, but may be affected by certain lifestyle or work related externalities such as smoking, alcohol or drug use, unhealthy diet, sedentary, hazardous or toxic work or living conditions, or the like. In those cases, patients may at the time be having no obvious signs or symptoms for cancer or precancer but may be trending towards cancer or precancer symptoms. However, the screening test, kit, and method as described can be used for general screening, for example, in patients that are not part of a high risk category, or are otherwise free from known cancer or precancer symptoms. Ideally, it would be beneficial to have the screening test, kit, and method as described to be part of an annual physical, or regular physician visits for patients in a target population, allowing for much earlier detection and intervention. As used herein, a target population can be generally people over the age of 30 years, but there is no reason why younger individuals could not be screened.

In particular embodiments, this invention provides a reliable screening tool for the detection of a wide variety of cancers, e.g., lungs, rectum, colon, lymph node, stomach, kidney, gallbladder, liver, pancreas, prostate, testes, breast, head and neck, cervix, uterus and ovaries. In precancerous conditions, for example, those in which the individual has no obvious signs or symptoms. In other words, the invention provides a tool which can detect cancer or precancer even when there is no indication that a condition exists. This also includes individuals who are genetically predisposed to certain cancers or who have a family history for such cancer (e.g., is in a "highly susceptible to subsequent cancer" category). However, the screening test, kit, and method as described can be used for general screening, for example, in patients that are not part of a high risk category, or are otherwise free from known cancer or precancer symptoms.

In some embodiments, when the screening tool of the invention shows evidence of a cancerous or precancerous condition, the invention further provides a step of beginning treatment for a cancerous or precancerous condition or reinstating a previously suspended treatment for a cancerous condition. In some embodiments, when the screening tool of the invention does not show evidence of a cancerous or precancerous condition, the invention provides a step of screening the individual again after a predetermined period of time.

In particular embodiments, the invention provides a screening tool for a variety of biological samples, biological matrices, and biological fluids. The language "biological sample" refers to any solution or extract containing a molecule or mixture of molecules that comprises at least one biomolecule that is subjected to extraction or analysis that originated from a biological source (such as, humans and animals). Biological samples are intended to include crude or purified, e.g., isolated or commercially obtained, samples. Biological sample may be, but are not limited to, inclusion bodies, biological fluids, biological tissues, biological matrices, embedded tissue samples, cells *(e.g.,* one or more types of cells), and cell culture supernatants. Particular examples may include blood plasma, urine, cerebrospinal fluid, synovial fluid and other biological fluids, including extracts of tissues, such as liver tissue, muscle tissue, brain tissue and heart tissue and the like.

The language "biological matrices" is intended to include anything that a cell contains or makes, *e.g.,* bone, inclusion bodies, blood components, cell debris, *e.g.,* cell lysates, etc.

The language "biological fluid" as used herein is intended to include fluids that are obtained from a biological source. Exemplary biological fluids include, but are not limited to, blood, blood plasma, urine, spinal fluid, mucosal tissue secretions, tears, interstitial fluid, saliva, sputum, synovial fluid, semen, and breast secretions. In specific embodiments, the biological fluid is a protein-containing or proteinaceous biological fluid. In still other embodiments, the biological fluid is sputum, saliva, nipple aspirate, prostatic massage secretion, pancreatic and bile duct fluid, rectal mucus or endocervical mucus. In particular embodiments, the biological fluid is not rectal mucus.

In detail, a marker carbohydrate or saccharide in a mucus or body fluid sample is detected by selective oxidation of the glycoprotein in the mucus or body fluid sample with galactose oxidase or comparable oxidant which will oxidize the primary hydroxy groups of only the galactose moieties in the saccharides present in the glycoprotein into aldehydic groups. The resulting aldehydic groups can then be visualized with a Schiff reagent, e.g., basic fuchsin which forms a magenta color.

The galactose moieties marker carbohydrates or saccharides are rapidly selectively oxidized at room temperature with galactose oxidase to aldehydic sugar moieties, e.g., in less than about 15 minutes, e.g., about 5-10 minutes, and even more rapidly at elevated temperatures, up to the deactivation temperature of the enzyme. The ratios of enzyme to substrate and vehicles suitable for activating the enzyme which are well known in the art when using this enzyme can be employed.

The oxidized sample, with or without first removal or inactivation of the galactose oxidase is then treated with a reagent which visualizes or permits visualization of the thus-produced aldehydic sugar moieties, e.g., fuchsin, rosaniline, magenta or other Schiff base decolorized dye.

The objects, features and advantages of the present invention are attained in one aspect thereof by providing a rapid, reliable with respect to false negatives and commercially feasible method for detecting the presence of a precancerous or cancerous condition in a human. The invention employs a test method which comprises obtaining a sample of mucus or body fluid, assaying the sample to detect the presence therein of at least one of the marker carbohydrates beta-D-Gal-(1->3)-D-GalNAc, Fuc-alpha-1->2 Gal-Beta(1->4)-Fuc-alpha-1->3-GlcNAc, Fuc-Alpha-1->2Gal-beta-(1->4)-Fuc-alpha-1->3-GlcNAc-beta-(1->3)-Gal-beta-(1>4)-GlcNAc and Fuc-alpha-1->2-Gal-beta-(1->4)-Fuc-alpha-1->3-GlcNAc-beta-(1->3)-Gal-beta-(1->4)-Fuc-alpha-1->3-GlcNAc; and, optionally, diagnosing the presence and degree of precancer or cancer based upon the amount of the marker carbohydrate(s) detected in the mucus or body fluid.

### Use of Galactose Oxidase Strip Test For Primary Screening

This technique uses the ability of galactose oxidase to selectively oxidize the C-6 hydroxyl group of galactose moieties of the glycoprotein in the mucus, e.g., both the galactose and N-acetyl galactosamine residues of the beta-D-Gal-(1->3)-D-GalNAc to D-galactohexodialdose. The presence of aldehyde sugar moieties in this thus-oxidized product is evidence of a marker carbohydrate in the glycoprotein. Their presence can be detected using Schiff's Reagent, e.g., basic fuchsin.

A sample is obtained and smeared on the scored side of a piece of membrane filter (e.g., Metricel membrane filter 0.45 µm, Gelman Sciences, Inc., Ann Arbor, Mich. 48106). An appropriate amount (depending on the size of the filter paper) of galactose oxidase is applied directly to the filter. After about 5-10 minutes reaction time, usually at room temperature, wash the membrane for 1 minute in deionized water and then place the membrane in Schiff's Reagent for 1 minute and then wash the membrane for 1 minute in running tap water. Shake off excess water and dry the membrane by air drying or in an oven. A bright magenta coloration of the mucus smear when completely dried indicates a positive test.

With typical results, no false negatives and far fewer false positives are found than in the conventional blood test for cancer of the colon. A sample giving a negative result is then tested in accordance with this invention for glycoprotein content by oxidation with periodic acid followed by treatment again with a Schiff's Reagent. Development of a magenta color confirms that the negative result is a biological negative and not a false negative due to sampling error. Because the whole procedure requires less than one-half hour, a second sample can conveniently be obtained from an individual whose first sample yielded a false negative.

### PREPARATION

### Storage-Stable Schiff Reagent Solution

Dissolve 1.0 gm of basic fuchsin in 200.0 ml of hot distilled water and bring to the boiling point. Cool to 50° C., add 20.0 ml of IN HCl and cool further and add 1.0 gm of sodium metabisulfate. Refrigerate in the dark until the solution becomes straw colored (about 48 hours). Then add 5 g of activated charcoal, thoroughly stir and remove the charcoal by filtration. The clear filtrate is a Schiff's Reagent which is storage-stable for many months, e.g., at least one year. Moreover, the magenta color which is produced therewith is more intense than that obtained with conventionally prepared Schiff s Reagent.

### Galactose Oxidase Test Strip

This disclosure describes a test strip or membrane with galactose oxidase pre-embedded. There is no requirement for a separate galactose oxidase solution. Galactose oxidase in the test strip or membrane may be encapsulated, which can be activated by moisture or in contact with water. The amount of galactose oxidase in the test strip or membrane is not limited, provided that the amount is sufficient to oxidize marker carbohydrates in the sample.

In one aspect, the test strip or membrane of this disclosure could be designed to have different amount of fluid intake to accommodate the different amount of marker carbohydrate in different mucus or body fluid. For example, a strip or membrane for testing rectal mucus could be different from a strip or membrane for testing secretions of breast in terms of fluid intake. In another aspect, the amount of galactose oxidase in the test strip or membrane can also be altered based on the concentrations of marker carbohydrate in different mucus or body fluid. In another aspect, the test strip or membrane further contains dry culture medium, and the dry culture medium can activate the galactose oxidase once water is added onto the test strip or membrane.

### Galactose Oxidase Test Strip Device

This disclosure describes a device with a test strip or membrane with galactose oxidase pre-embedded and a container with Schiff reagent solution, wherein the Schiff reagent solution is not initially in contact with the test strip or membrane, and can be activated to contact the test strip or membrane after the marker carbohydrate is oxidized by galactose oxidase.

The mechanism to activate the Schiff reagent solution (to contact the test strip or membrane after the marker carbohydrate is oxidized by galactose oxidase) is not limited. In one aspect, the mechanism can be a twist valve, a breakable barrier, or the like, wherein after open the twist valve or breaking the barrier, the Schiff reagent solution contacts the test strip or membrane. In another aspect, the mechanism to activate the Schiff reagent solution is to manually transfer the Schiff reagent solution onto the test strip or membrane. In one aspect, the device of the present invention includes all the components in a single unit to minimize the sampling or procedural error.

In one aspect, the test strip or membrane with galactose oxidase pre-embedded further contains dry culture medium, wherein the dry culture medium can activate the galactose oxidase once water is added onto the test strip or membrane. In another aspect, the pre-embedded galactose oxidase in the test strip or membrane is microencapsulated. In yet another aspect, the pre-embedded galactose oxidase in the test strip or membrane can be activated by adding a few drops of water on the test strip or membrane, or by adding the mucus or body fluid onto the test strip or membrane.

In one aspect, this disclosure describes the use of the device to screen a cancerous or precancerous condition. The type of cancerous or precancerous condition is not limited, giving that the marker carbohydrates present in the mucus or body fluid of the cancerous or precancerous condition, for example, rectal, colon, blood, lymph node, stomach, kidney, gall bladder, prostate, testes, breast, cervix and ovaries.

In certain embodiments, the test strip or membrane with a galactose oxidase pre-embedded further contains a pre-embedded Schiff reagent. In such embodiments, the pre-embedded galactose oxidase is activated prior to activation of the pre-embedded Schiff reagent such that reaction of the sample with galactose oxidase happens prior to reaction with the Schiff reagent.

### Galactose Oxidase Test Strip with Embedded Schiff Reagent

Described herein is a test strip or membrane for reaction of a sample with galactose oxidase with the Schiff reagent pre-embedded. The Schiff reagent in the test strip or membrane may be encapsulated, which can be activated by swelling with moisture or by contact with a pore-forming agent. The amount of Schiff reagent in the test strip or membrane is not limited, provided that the amount is sufficient to react with marker carbohydrates in the sample after oxidation with galactose oxidase,
In one aspect, the test strip or membrane of this disclosure could be designed to have different amount of fluid intake to accommodate the different amount of marker carbohydrate in different mucus or body fluid. For example, a strip or membrane for testing rectal mucus could be different from a strip or membrane for testing secretions of breast in terms of fluid intake. In another aspect, the amount of galactose oxidase in the test strip or membrane can also be altered based on the concentrations of marker carbohydrate in different mucus or body fluid. In another aspect, the test strip or membrane further contains dry culture medium, and the dry culture medium can activate the galactose oxidase once water is added onto the test strip or membrane.

### Galactose Oxidase Test Strip Device with embedded Schiff reagent

Described herein is a device with a test strip or membrane with a Schiff reagent pre-embedded and a container with galactose oxidase reagent solution, wherein the galactose oxidase reagent solution is not initially in contact with the test strip or membrane. The sample and the galactose oxidase reagent solution is added to the test strip and allowed to react prior to activation of the Schiff reagent.

In one aspect, the test strip or membrane with a Schiff reagent pre-embedded further contains dry culture medium, wherein the dry culture medium can activate once a sample is added onto the test strip or membrane. In another aspect, the pre-embedded Schiff reagent in the test strip or membrane is microencapsulated. In yet another aspect, the pre-embedded Schiff reagent in the test strip or membrane can be activated by adding a few drops of water on the test strip or membrane, or by adding the mucus or body fluid onto the test strip or membrane. In another aspect, the pre-embedded Schiff reagent in the test strip can be activated by adding a pore-forming agent to the strip before, after or concurrently with the addition of the sample.

In one aspect, this disclosure is directed to the use of the device to screen a cancerous or precancerous condition. The type of cancerous or precancerous condition is not limited, giving that the marker carbohydrates present in the mucus or body fluid of the cancerous or precancerous condition, for example, rectal, colon, blood, lymph node, stomach, kidney, gall bladder, prostate, testes, breast, cervix and ovaries.

In certain embodiments, the test strip or membrane with a Schiff reagent pre-embedded further contains a pre-embedded galactose oxidase. In such embodiments, the pre-embedded galactose oxidase is activated prior to activation of the Schiff reagent such that reaction of the sample with galactose oxidase happens prior to reaction with the Schiff reagent.

### Embedding and Encapsulation

As discussed above, in certain embodiments, the galactose oxidase is pre-embedded into the test strip or membrane. In some embodiments, the galactose oxidase is embedded in, absorbed into, coated on, or impregnated directly into the membrane or test strip. In particular embodiments, the galactose oxidase is encapsulated prior to being embedded, absorbed, coated or impregnated into the membrane or test strip.

In embodiments where the galactose oxidase is encapsulated or microencapsulated, the encapsulating materials may be made with one or more polymers to provide a controlled, sustained, or immediate release of the reagents.

As discussed above, in certain embodiments, the galactose oxidase is pre-embedded into the test strip or membrane. In some embodiments, the Schiff reagent is embedded in, absorbed into, coated on, or impregnated directly into the membrane or test strip. In particular embodiments, the Schiff reagent is encapsulated prior to being embedded, absorbed, coated or impregnated into the membrane or test strip.

In embodiments where the Schiff reagent is encapsulated or microencapsulated, the encapsulating materials may be made with one or more polymers to provide a controlled, sustained, or immediate release of the reagents.

In certain embodiments, encapsulating material may be prepared following the method of Caruso (Phys. Chem. Chem. Phys., 2011,13, 4782); Sukhishvili (Chem. Mater., 2006, 18 (2), 328); US20150164805; EP2213280; or Schwendeman (J Control Release. 2014;196:60). Materials used to prepare encapsulating material include (but are not limited to): emulsifiers, materials with varied melting points, materials with different hydrophilic/lipophilic balances (HLB), phospholipids, fatty acids, plant sterols, sorbitan esters, bees wax, carnauba wax, paraffin, stearates, shellac, cellulose derivatives, maltodextrin, starch, gums, cellulose, Polypyrrole, polycarbonate, cetyltrimethylammonium halides, silanes, diblock copolymers, triblock copolymers such as poly(ethylene oxide)-blockpoly(propylene oxide)-block-poly(ethylene oxide), named P123 (PEO20PPO70PEO20) and F127 (PE0106PP070-PE0106), alginate, chitosan, xanthan gum, polysaccharide, polysaccharide hydrogel, poly(lysine), poly(acrylic acid), agarose, PEG, poly(hydroxyethylmetacrylate-methyl methacrylate), poly(acrylic acid-co-acrylamide), poly(allylaminehydrochloride), poly(styrenesulfonate sodium salt), poly-(diallyldimethylammonium chloride), poly(ethylene imine), N-hydroxysuccinimide-PEG, maleimide-PEG-conjugated phospholipids, paraffin, cyclodextrin, carboxymethylated polysaccharide, polycaprolactone, humic substances, Span 60, cholesterol, N-trimethyl chitosan chloride, poly(methyl methacrylate), poly(2-hydroxyethyl methacrylate), poly(N-isopropylacrylamide), poly(N-isopropylmethacrylamide), poly(N-n-propylacrylamide), carboxymethylcellulose, plastic, gold, molecular weight cut-off filters, hybrid organic/inorganic materials, metal oxides, plastics, silica including SBA-15 (PD 50-89Å) and MCM-41, ceramics, clays, smectic clays, and niosomes.

In certain embodiments, the encapsulating material contain, or are subsequently modified to display, a functional group that is capable of subsequently reacting with the membrane or test strip using standard synthetic reactions. For example, in certain embodiments, the encapsulating material may contain an amino-alkyl functional group, an ester functional group, an amide functional group, or a carbamate function group which may be reacted with an active group on the membrane or test strip to provide immobilization of the reagent. There are a number of standard coupling methods known in the literature, including but not limited to March (Advanced Organic Chemistry, 3rd Edition, Wiley, New York, 1985); Odian (The Principles of Polymerization, 2nd Edition, Wiley, New York, 1981); and Bioconjugate Techniques (Hermanson, G. T., Bioconjugate Techniques; Academic Press: San Diego, 1996.

Other methods of encapsulation include, but are not limited to, those disclosed in US20160075976A1 or US6258870B1.

### Polymeric Materials

Suitable thermoplastic polymers for incorporation as the encapsulation material include, but are not limited to polylactides, polyglycolides, polycaprolactones, polyanhydrides, polyamides, polyurethanes, polyesteramides, polyorthoesters, polydioxanones, polyacetals, polyketals, polycarbonates, polyorthocarbonates, polyphosphazenes, polyhydroxybutyrates, polyhydroxyvalerates, polyalkylene oxalates, polyalkylene succinates, poly(malic acid) polymers, polymaleic anhydrides, poly(methylvinyl) ethers, poly(amino acids), chitin, chitosan, and copolymers, terpolymers, or combinations or mixtures of the above materials.

Examples of biodegradable polymers and oligomers suitable for use in the compositions and methods of the present invention include, but are not limited to: poly(lactide)s; poly(glycolide)s; poly(lactide-co-glycolide)s; poly(lactic acid)s; poly(glycolic acid)s; and poly(lactic acid-co-glycolic acid)s; poly(caprolactone)s; poly(malic acid)s; polyamides; polyanhydrides; polyamino acids; polyorthoesters; polyetheresters; polycyanoacrylates; polyphosphazines; polyphosphoesters; polyesteramides; polydioxanones; polyacetals; polyketals; polycarbonates; polyorthocarbonates; degradable polyurethanes; polyhydroxybutyrates; polyhydroxyvalerates; polyalkylene oxalates; polyalkylene succinates; chitins; chitosans; oxidized celluloses; and copolymers, terpolymers, blends, combinations or mixtures of any of the above materials.

As used herein, "hydrophobic" refers to a polymer that is substantially not soluble in water. As used herein, "hydrophilic" refers to a polymer that may be water-soluble or to a polymer having affinity for absorbing water, but typically not when covalently linked to the hydrophobic component as a co-polymer, and which attracts water into the device.

Hydrophilic polymers suitable for use herein can be obtained from various commercial, natural or synthetic sources well known in the art. Suitable hydrophilic polymers include, but are not limited to: polyanions including anionic polysaccharides such as alginate; agarose; heparin; polyacrylic acid salts; polymethacrylic acid salts; ethylene maleic anhydride copolymer (half ester); carboxymethyl amylose; carboxymethyl cellulose; carboxymethyl dextran; carboxymethyl starch; carboxymethyl chitin/chitosan; carboxy cellulose; 2,3-dicarboxycellulose; tricarboxycellulose; carboxy gum arabic; carboxy carrageenan; carboxy pectin; carboxy tragacanth gum; carboxy xanthan gum; carboxy guar gum; carboxy starch; pentosan polysulfate; curdlan; inositol hexasulfate; beta.-cyclodextrin sulfate; hyaluronic acid; chondroitin-6-sulfate; dermatan sulfate; dextran sulfate; heparin sulfate; carrageenan; polygalacturonate; polyphosphate; polyaldehydo-carbonic acid; poly-1-hydroxy-1-sulfonate-propen-2; copolystyrene maleic acid; mesoglycan; sulfopropylated polyvinyl alcohols; cellulose sulfate; protamine sulfate; phospho guar gum; polyglutamic acid; polyaspartic acid; polyamino acids; and any derivatives or combinations thereof. One skilled in the art will appreciate other hydrophilic polymers that are also within the scope of the present invention.

Various water-soluble polymers suitable for use herein include, but are not limited to: poly (alkyleneglycol), polyethylene glycol ("PEG"); propylene glycol; ethylene glycol/propylene glycol copolymers; carboxylmethylcellulose; dextran; polyvinyl alcohol ("PVOH"); polyvinyl pyrolidone; poly (alkyleneamine)s; poly (alkyleneoxide)s; poly-1,3-dioxolane; poly-1,3,6-trioxane; ethylene/maleic anhydride copolymers; polyaminoacids; poly (n-vinyl pyrolidone); polypropylene oxide/ethylene oxide copolymers; polyoxyethylated polyols; polyvinyl alcohol succinate; glycerine; ethylene oxides; propylene oxides; poloxamers; alkoxylated copolymers; water soluble polyanions; and any derivatives or combinations thereof. In addition, the water-soluble polymer may be of any suitable molecular weight, and may be branched or unbranched.

Depending on the desired softness and flexibility of the encapsulation material, the rate and extent of reagent release, rate of degradation, and the like, the amount and type of polymer can be varied to produce the desired result.

### Encapsulation Shells and Impregnated layers

In certain embodiments, the polymers form an encapsulation shell which may be rendered porous under certain conditions and over time, thereby controlling the release. The pores can be formed by a swelling of the polymer shell or by a dissolution or degradation of the shell.

The mass, volume and thickness of the polymers in each encapsulation shell/sphere can also be varied to adjust the release rate of the incorporated reagent.

The use of the term shell/sphere, as used herein, is not limiting as to the shape of the encapsulation material. Although the shape of the material is generally spherical, it is possible to prepare and utilize conical shells, tubular shells, oblong shells, cylindrical rods, and the like. In certain embodiments the material may be amorphous or irregularly shaped. In certain other embodiments the encapsulation material may be coated or bonded to the surface of a scaffolding material or a chromatographic material. In such embodiments, the encapsulation material may take the shape of the material to which it is bonded. In certain embodiments in which the chromatographic material or scaffolding material is porous, the encapsulation may or may not penetrate the pores of the underlying material.

In certain other embodiments, the polymers may be impregnated with the reagent and coated onto the surface of a membrane or test strip. In such embodiments, the reagent is blended or mixed with the polymers such that the reagent becomes embedded, encapsulated or impregnated into the polymer matrix. In such embodiments, the encapsulation material does not form a discreet encapsulation shell but, instead, the encapsulation material containing the reagent may be coated onto a membrane or test strip as a layer and, optionally, covalently or ionically bonded thereto.

In certain embodiments, the encapsulation material may be a wax, hydrogel, a silicone rubber, or a trehalose glass. The use of hydrogels, silicone rubbers and trehalose glasses is particularly suited for the impregnation of reagents into the polymer material though any suitable polymer may be used in such embodiments.

In still other embodiments, the reagent can be loaded into the pores of a porous membrane or test strip material. In such embodiments, once the reagent is loaded into the pores of the porous material, the porous material can be coated with one or more polymeric encapsulation materials as described herein.

### Inducing release

The encapsulated galactose oxidase reagent may be released from the encapsulating materials by any number of means as may be known to one of ordinary skill in the art. In certain embodiments of the invention, such release can be induced by contacting the encapsulating material with a pore-forming agent. In other embodiments of the invention, the release can be induced by a physical change or a chemical change. For example, and without limitation, the release can be induced by changes in temperature, pH, ionic charge, counterion charge, or counterion atom. Similarly, the release can be induced by contacting the encapsulating material with a solvent including, but not limited to, an organic solvent, an aqueous solvent, an aliphatic solvent, an aromatic solvent, an oxygenated solvent, or a halogenated solvent, or water.

In general, the release rate for a reagent will be determined by the skilled artisan based on the membrane or test strip being utilized. In certain embodiments, the desired release rate is immediate whereas in other embodiments the release rate is controlled so that the reagent is released over a period of time. In certain embodiments, the reagent is released over the course of the testing/workflow such that about 100% of the reagent is released by the time that about 100% of the sample has been introduced. In other embodiments, the reagent is released over the course of the testing/workflow such that about 100% of the reagent is released by the time that about 90% of the sample has been introduced; by the time that about 80% of the sample has been introduced; by the time that about 75% of the sample has been introduced; by the time that about 50% of the sample has been introduced; or by the time that about 25% of the sample has been introduced.

### Pore-Forming Agents

Other additives can be used to advantage in further controlling the desired release rate of a reagent for a particular testing/workflow protocol. For example, if the thermoplastic polymer liquid composition is too impervious to water, a pore-forming agent can be added to generate additional pores in the matrix. Any compatible water-soluble material can be used as the pore-forming agent. These agents can be either soluble in the liquid composition or simply dispersed within it. They are capable of dissolving, diffusing or dispersing out of both the coagulating polymer matrix and the formed polymer system whereupon pores and microporous channels are generated in the matrix and system. The amount of pore-forming agent (and size of dispersed particles of such pore-forming agent, if appropriate) within the composition will directly affect the size and number of the pores in the polymer system.

Other factors can also influence the size and/or diameter of the pores formed in the polymer system. For example, the amount of organic solvent, and the rate at which the polymer system solidifies, can all affect the porosity of the polymer system. Although a generally microporous matrix without a resolved core and skin can be produced according to the invention, typically, without an additional pore-forming agent a polymer system formed from the liquid composition is composed of a surface skin and inner core. The surface skin is typically less porous, and even relatively nonporous, when compared to the inner core. The inner core can contain pores with a diameter of about 10-1000 um. With additional pore-forming agent, the pore sizes of the core and skin become substantially uniform such that they both have pores in the range of 10 to 1000 um.

The concentration of pore-forming agent relative to thermoplastic polymer in the composition will vary according to the degree of pore-formation desired. Generally, this concentration will range from about 0.01 to 1 gm of pore-forming agent per gm of polymer. If the agent is soluble in the liquid composition, then the mixing or distribution of the agent in the liquid composition and the aggregation when the thermoplastic coagulates will determine the size of the resultant pores as the agent dissolves out of the polymer matrix.

Pore-forming agents include, any pharmaceutically acceptable organic or inorganic substance that is substantially miscible in water and body fluids and will dissipate from the forming and formed matrix into aqueous medium or body fluids or water-immiscible substances that rapidly degrade to water-soluble substances. The pore-forming agent may be soluble or insoluble in the polymer liquid composition of the invention. In the liquid composition of the invention, it is further preferred that the pore-forming agent is miscible or dispersible in the organic solvent to form a uniform mixture. Suitable pore-forming agents include, for example, sugars such as sucrose and dextrose, salts such as sodium chloride and sodium carbonate, and polymers such as hydroxylpropylcellulose, carboxymethylcellulose, polyethylene glycol, and polyvinylpyrrolidone. The size and extent of the pores can be varied over a wide range by changing the molecular weight and percentage of pore-forming agent incorporated into the polymer system.

Other excipient materials can be added to the devices to alter porosity, for example, materials like sucrose, dextrose, sodium chloride, sorbitol, lactose, polyethylene glycol, mannitol, fructose, polyvinyl pyrrolidone or appropriate combinations thereof. Additionally, the active agents may be dispersed with oils (e.g., sesame oil, corn oil, vegetable), or a mixture thereof with a phospholipid (e.g., lecitin), or medium chain fatty acid triglycerides (e.g., Miglyol 812) to provide an oily suspension.

### Testing method

This disclosure provides an in vitro method for rapidly detecting the expression of a marker carbohydrate in a subject using a test strip or membrane with galactose oxidase pre-embedded and Schiff reagent solution, or with galactose oxidase and a pre-embedded Schiff reagent.

This disclosure also provides an in vitro method for rapidly testing a human being for a cancerous or precancerous condition using a test strip or membrane with galactose oxidase pre-embedded and Schiff reagent solution, or with galactose oxidase and a pre-embedded Schiff reagent.

In one aspect, the method contains the steps of applying mucus or body fluid to a test strip or membrane with galactose oxidase pre-embedded, oxidizing marker carbohydrates in the mucus or body fluid that contains marker carbohydrates by reacting with galactose oxidase, and activating a container with Schiff reagent solution adjacent to the test strip or membrane to contact the test strip or membrane, and wherein the Schiff reagent solution is not initially in contact with the test strip or membrane.

In one aspect, the method contains the steps of reacting mucus or body fluid with galactose oxidase and contacting the oxidized sample to a test strip or membrane with a Schiff reagent pre-embedded, and activating the Schiff reagent solution in the test strip or membrane to contact the sample.

In some embodiments, the method contains one or more steps selected from: applying water onto the test strip or membrane before applying mucus or body fluid to the test strip or membrane, removing the mucus or body fluid by washing before activating a container with Schiff reagent solution, washing the test strip or membrane with tap water after letting the Schiff reagent solution to contact the test strip or membrane, drying the test strip or membrane, and/or evaluating color of the test strip or membrane.

In some embodiments, the method contains one or more steps selected from: applying water onto the test strip or membrane before applying mucus or body fluid to the test strip or membrane, removing the mucus or body fluid by washing before activating the Schiff reagent, washing the test strip or membrane with tap water after letting the Schiff reagent contact the sample, drying the test strip or membrane, and/or evaluating color of the test strip or membrane.

In one aspect, the period to oxidize marker carbohydrates in the mucus or body fluid by reacting with galactose oxidase is not particular limited. For example, the period of oxidation can be 3 to 30 mins, 5 to 20 mins, 7 to 15 mins, or 8 to 12 mins. In another aspect, the period to let the Schiff reagent solution to contact the test strip or membrane is not particular limited. For example, the period of contacting can be 0.2 to 10 mins, 0.5 to 5 mins, 0.8 to 3 mins, or 1 to 2 mins.

In another aspect, the mechanism to activate the Schiff reagent solution (to contact the test strip or membrane after the marker carbohydrate is oxidized by galactose oxidase) is not limited. In one aspect, the mechanism can be a twist valve, a breakable barrier, or the like, wherein after open the twist valve or breaking the barrier, the Schiff reagent solution contacts the test strip or membrane. The materials for the breakable barrier is not limited, and the breakable barrier can be made from plastic, glass, or any materials that is suitable for a liquid container.

### Testing kit

The simple use test kit is packaged in a conventional manner in a cardboard carton containing (a) a capped vial containing an amount of storage-stable basic fuchsin, prepared according to the preparation hereinafter, sufficient to saturate twice (b) a strip of membrane filter (Metricel membrane filter 0.46 µm, Gelman Sciences, Inc., Ann Arbor, Michigan). Also present in the kit is (c) an amount of a storage-stable form of galactose oxidase which is present in the kit in a sealed capped bottle impregnated in the strip of membrane filter in an amount sufficient to oxidize marker carbohydrates in the sample. Also present are (d) periodic acid, and (e) a color chart for comparison with the test result and interpretation thereof.

For field testing purposes, the kit contains a plurality of the membrane filters strips, e.g., 5, 10, 50, 100 or more and the amounts of galactose oxidase, buffer solution and basic fuchsin solutions are increased proportionately.

This disclosure provides a screening kit for rapidly detecting the expression of a marker carbohydrate in a subject using a test strip or membrane with galactose oxidase pre-embedded and Schiff reagent solution.

This disclosure further describes a screening kit for rapidly testing a human being for a cancerous or precancerous condition containing a test strip or membrane with galactose oxidase pre-embedded and a container with Schiff reagent solution.

In one aspect, the test strip or membrane with galactose oxidase pre-embedded further contains dry culture medium, wherein the dry culture medium can activate the galactose oxidase once water is added onto the test strip or membrane. In another aspect, the pre-embedded galactose oxidase in the test strip or membrane is microencapsulated.

This disclosure also describes a screening kit for rapidly detecting the expression of a marker carbohydrate in a subject using a test strip or membrane with galactose oxidase and a pre-embedded Schiff reagent.

This disclosure further describes a screening kit for rapidly testing a human being for a cancerous or precancerous condition containing a test strip or membrane with galactose oxidase and a pre-embedded Schiff reagent.

In one aspect, the pre-embedded Schiff reagent in the test strip or membrane is microencapsulated.

In one aspect, this disclosure describes the use of the testing kit to screen a cancerous or precancerous condition. The type of cancerous or precancerous condition is not limited, giving that the marker carbohydrates present in the body fluid of the cancerous or precancerous condition, for example, rectal, colon, blood, lymph node, stomach, kidney, gall bladder, prostate, testes, breast, cervix and ovaries cancerous or precancerous condition.

### Populations showing no obvious signs or symptoms of a cancerous or precancerous condition

In particular embodiments, the invention describes a tool for monitoring individuals who had have a cancer and underwent treatment, for recurrence of the disease. A study has shown 32 of 53 (60%) colon cancer patients being positive for the marker after tumor resection. Five out of these 32 (16%) with post-operative persistence of the positivity had tumor recurrence within a year. Thus, monitoring post-operative patients with the marker and the test will allow physicians to identify recurrent cancer at an early stage, and appropriate management instituted.

In another embodiment in 28 individuals who had no signs and symptoms of lung cancer, 21 were found to be positive of which 15 patients were identified to have lung cancer on further work-up. Thus, monitoring individuals with positive test is of great importance in identifying the disease at a very early stage.

Comparative and correlative studies of the pathology and pathogenesis of colon cancer in animal models and human disease have resulted in conceptualization of 'field effect" theory and identification of markers that are expressed early during carcinogenesis. This assimilated body of knowledge has resulted in development of a simple rectal mucus test for colon cancer screening which can also be adapted for screening other epithelial cancers. The marker galactose-N acetylgalactosamine (Gal-GalNAc) is expressed in the rectal mucus of patients with colonic cancer or precancerous lesions and is detected by enzymatic oxidation (10 minutes) followed by color reaction (1 minute). The high sensitivity, specificity, positive predictive value and negative predictive value, as well as the cost-effectiveness of this test makes it a great tool in our strategies for early detection, hence control of colon cancer. Similar expression of this marker in cancers of breast, lungs, prostate, uterus, pancreas, makes it a potentially useful general cancer screening test. Because of its high accuracy (e.g., as opposed to the fecal occult blood tests) and cost-effectiveness (e.g., compared to chest X-ray) it would reduce the number of unnecessary colonoscopies and other expensive procedures, thereby decreasing the total national health-care cost to the society.

The expression of Gal-GalNAc was determined in a total of 133 tissue samples from 81 cases of the carcinomas of the breast, ovary, pancreas, stomach, and endometrium and 52 cases of respective normal controls. None of the 52 cases of normal tissues (except 15 cases of stomach) showed expression of Gal-GalNAc. In contrast, 100% of adenocarcinomas from the breast (19 of 19), ovary (15 of 15), and pancreas (6 of 6), and 94.1% of stomach (16 of 17) cancers, and 91.7% (11 of 12) of uterine adenocarcinomas expressed Gal-GalNAc. Additionally, 62 of 65 (95.4% sensitivity) cancers of the prostate expressed the marker, as opposed to none of the 35 benign hyperplasia of prostate (100% specificity). The normal epithelia and their secretions in the vicinity of the carcinoma (within the "field") in the breast, bronchus, endometrium, and pancreatic duct also expressed Gal-GalNAc in contrast to normal tissues obtained from noncancerous individuals, which were totally nonreactive. Thus, it was concluded that the tumor marker Gal-GalNAc recognized by Galactose Oxidase- Schiff sequence was highly expressed not only by a variety of adenocarcinomas but also by the apparently normal-appearing epithelia and their secretions in the vicinity of, or even away from cancers.

Adenocarcinomas are mucus producing tumors. Surprisingly, it has been found that the marker is expressed also in the coughed-up sputum of individuals harboring cancers that are not adenocarcinomas; these cancers e.g., squamous cell carcinoma, small cell undifferentiated carcinoma (aka oat cell carcinoma), large cell undifferentiated carcinoma, etc. do not produce mucus. The presence of the marker in the sputum samples of these non-mucus secreting cancers would not be expected or appreciated by those having ordinary skill in the art.

In a study 113 of 123 (91.9%) of patients with squamous cell cancer and 16 of 19 (84.2%) patients with small cell undifferentiated cancers were unexpectedly expressing the marker in coughed-up sputum.

### Expression of Gal-GalNAc by various types of cancers of the lung:

| **Diagnosis** | **Patients expressing the marker/total (%)** |
|---|---|
| Squamous cell carcinoma | 113/123 (91.9%) |
| Adenocarcinoma | 36/40 (90%) |
| Small cell (oat cell) undifferentiated carcinoma | 16/19 (84.2%) |

Likewise, the cancers of the head & neck region include a very large proportion of non-mucus producing squamous cell carcinomas besides the mucus-producing adenocarcinomas. The saliva in those people harboring a cancer or precancerous lesion will be expressing the marker.

With respect to the kit aspect described herein, it was discovered, surprisingly, that if the generally accepted technique for producing the Schiff's Reagent of mixing the reagent after preparation with activated charcoal and then refrigerating is not followed, a solution which is storage-stable for months at room temperature can easily and reproducibly be produced. This method renders the color reaction more intense than is obtained with conventional Schiff's Reagent. According to the technique of this invention, the Schiff's base is first refrigerated in the absence of activated charcoal, e.g., at about 0°-15° C., preferably about 0°-10° C., until the color thereof fades to a straw shade, e.g,. for 1, 2 or more days usually about 48 hours. After the solution has faded to a straw color, it is then treated with activated charcoal or like surface-active absorbent, e.g., with stirring, e.g., at room temperature for from about a few minutes to several hours or days. After removing the charcoal, e.g., by filtration, the Schiff's Reagent is filled into a vial or bottle of a volume suitable for conducting the number of tests for which the kit is designed, e.g., 1, 5, 10, 50, 100 or more. While the screening methods and kit disclosed herein is described using Schiff's Reagent, it should be appreciated by those having ordinary skill in the art that in some embodiments, materials that function substantially the same or in a similar manner as Schiff's Reagent may also be used for staining, dyeing, or otherwise marking samples without departing from the scope of this disclosure.

In another aspect described herein, the detection method is used to field test a plurality of individuals for any cancerous or precancerous condition. When conducting the steps thereof, preferably only those samples which assay negative for a marker saccharide is further tested for any saccharide. Alternatively, each sample can be divided into two portions, the first of which is assayed for a marker saccharide and the latter is assayed for any other saccharide oxidizable to an aldehydic sugar moiety, thereby permitting the diagnostic procedure and the false negative tests to be conducted concurrently and thereby further shortening the time period required to be able to report a biologically negative test result.

With further respect to the speed aspect of this invention, the assay for a marker saccharide can be conducted far more rapidly than was apparent from U.S. Pat. No. 4,857,457 and in my publication in Human Pathology, supra, as evident from the Example hereinafter, the whole testing procedure including the test for false negatives, can be completed in less than one-half hour, e.g., within about 15-20 minutes after collection of the sample. This enables the testing physician or laboratory to report the results of the test (or take another sample if a biological negative result is obtained) before the individual leaves the testing area.

A marker saccharide in a sample is detected by selective oxidation of the glycoprotein in the mucus sample with galactose oxidase or comparable oxidant which will oxidize the primary hydroxy groups of only the galactose moieties in the saccharides present in the glycoprotein into aldehydic groups. The resulting aldehydic groups can then be visualized with a Schiff's Reagent, e.g., basic fuchsin which forms a magenta color. The sample can be rectal mucus, coughed-up sputum, nipple aspirate, endocervical mucus, prostatic massage secretion, pancreatic duct or bile duct secretions.

The galactose moieties marker saccharides are rapidly selectively oxidized at room temperature with galactose oxidase to aldehydic sugar moieties, e.g., in less than about 15 minutes, e.g., about 5-10 minutes, and even more rapidly at elevated temperatures, up to the deactivation temperature of the enzyme. The ratios of enzyme to substrate and vehicles suitable for activating the enzyme which are well known in the art when using this enzyme can be employed.

The oxidized sample, with or without first removal or inactivation of the galactose oxidase is then treated with a reagent which visualizes or permits visualization of the thus-produced aldehydic sugar moieties, e.g., fuchsin, rosaniline, magenta or other Schiff base decolorized dye.

The marker carbohydrates can be assayed by selective oxidation of a galactose moiety therein with galactose oxidase followed by visualization of the thus-produced aldehydic saccharide with basic fuchsin. They can also be assayed employing an agglutination inhibition test, e.g., using an approximately stoichiometric amount of peanut agglutinin, Jacalin, wheat germ agglutinin or other suitable lectins by detecting the presence of biotinylated lectin and detecting the complex by reacting it with avidin which is conjugated another enzyme such as horseradish peroxidase, alkaline phosphatase etc. Similarly the marker can be detected using a primary antibody against the marker, followed by reaction with a secondary antibody conjugated with an enzyme and detected by a dye according the standard ELISA assay.

A marker saccharide in a biological fluid sample can also be detected by agglomeration of sensitized beads.

The objects, features and advantages of the present invention are attained in one aspect thereof by providing a rapid, reliable with respect to false negatives and commercially feasible method for detecting the presence of a precancerous or cancerous condition in a human. The invention employs a test method which comprises obtaining a sample of rectal mucus, assaying the sample to detect the presence therein of at least one of the marker carbohydrates beta-D-Gal-(1->3)-D-GalNAc, Fuc-alpha-1->2 Gal-Beta(1->4)-Fuc-alpha-1->3-GlcNAc, Fuc-Alpha-1->2 Gal-beta-(1->4)-Fuc-alpha-1->3-GlcNAc-beta-(1->3)-Gal-beta-(1>4)-GlcNAc and Fuc-alpha-1->2-Gal-beta-(1->4)-Fuc-alpha-1->3-GlcNAc-beta-(1->3)-Gal-beta-(1->4)-Fuc-alpha-1->3-GlcNAc; and, optionally, diagnosing the presence and degree of precancer or cancer based upon the amount of the marker carbohydrate(s) detected in the rectal mucus. Except for the modifications thereof of this invention which renders the test procedure rapid, reliable as to false negatives and commercially feasible, the test methods are those disclosed in U.S. Pat. No. 4,857,457 and prior filed U.S. patent application Ser. No. 07/228,468, filed Aug. 5, 1988.

In one embodiment, the assay may be performed by reacting the body fluid with a precise amount of peanut agglutinin or other specific binding moiety such as lectins (PNA, JAC, ACA, SNA, SRL, WGA, Galectin-3, and the like), and/or any suitable antibodies for the saccharide and then detecting the presence of unbound moiety. The reactant moiety can be immobilized onto a water-insoluble support, such as a membrane filter or solid beads of latex, plastic, glass, etc. In order to increase the sensitivity of the method, the reactant moiety can first be biotinylated in a conventional manner.

The complex can be detected by any of various suitable techniques, either directly or indirectly, e.g., immunologically, enzymatically, oxidative-reductively etc. Presently, preferred is the formation of a complex with avidin conjugated to a suitable marker, e.g., fuchsin or other dyes, radioactive labelling, fluorescent dyes such as fluorescein isothiocyanate or Rhodamine B, luminescent dyes such as luciferol, luminol, biotin, etc.

The presence of the disaccharide beta-D-Gal-(1->3)-GalNAc is readily detected by agglomeration of sensitized beads which have been coated with PNA, JAC, SRL, WGA, ACA, SNA, Galactin-3 etc., e.g., glass, agarose, polystyrene, latex, etc. A preferred method for detecting the presence of the complex is by selectively oxidizing the galactose moiety of the saccharide to an aldehydic disaccharide, e.g., with galactose oxidase, and detecting the presence of the thus-oxidized aldehydic sugar moiety.

In one aspect, the assay test detects specific biochemical changes in a subject associated with a cancerous or precancerous condition, e.g., of the large intestine, which results in the production of the disaccharide beta-D-Gal-(1->3)-D-GalNAc, also known as T-antigen or TF-antigen, which is absent in the body fluids of normal individuals but is present in the rectal mucus of individuals with at least some cancerous and precancerous conditions. Shamsuddin et al. developed various techniques for the detection of this sugar moiety in a simple and inexpensive manner (but not as rapidly as the method employed in this invention or which permits the use of storage-stable fuchsin or which eliminates false negatives), which techniques can be used to screen individuals for large intestinal diseases generally, including cancer.

The lectin, peanut agglutinin (PNA) specifically binds with T-antigen and causes agglutination of T-antigen activated RBC. Exploiting these characteristics of PNA, initially a simple inhibition assay has been developed wherein T-antigen in a body fluid sample will bind with PNA and, therefore, PNA will not react with RBC and the red cells will accordingly not agglutinate. This test is very simple and can be performed rapidly. Using microtiter plates, a large number of samples can be screened in a short time. The galactose oxidase test can be done conveniently on a strip of membrane filter.

Because not all cancerous and precancerous conditions generate all of the marker carbohydrates identified herein in the same proportions, in one embodiment of this invention more than one of the assay tests described herein is used to test a plurality of samples of proteinaceous bodily fluids of an individual being screened for a specific cancerous or precancerous condition.

In addition to cancerous conditions, the tests used in methods of this invention can detect other diseases of the colon including those that carry a high risk of cancer such as polyps, fistula, Crohn's disease, and ulcerative colitis.

In one embodiment, the biological fluid is tested for the marker carbohydrates as first step screening test for the presence of any cancerous or precancerous condition. If the screening test is positive, other proteinaceous body secretions easily accessed by non-invasive methods would be tested as a second step as a screening method for targeting a cancer or precancer in a specific organ.

Other properties, such as immobilization of PNA or other suitable lectins onto a water-insoluble support, immunological detection of the glycoconjugate or oxidation of the sugar moiety and detection by dyes, radio-chemicals, etc., can be exploited to develop additional assays. The use of an antibody directed against this sugar moiety in an immunoassay enables accurate estimation and monitoring of this moiety in rectal mucus as well as other body fluids. In the immunoassay, the antibody can be tagged by a radioactive fluorescent or other suitable label for quantitative or semiquantitative detection.

Avidin, a glycoprotein (67,000 MW), has an extraordinarily high affinity for the vitamin biotin. Inasmuch as biotin molecules can be coupled to various proteins (biotinylation), avidin can be conjugated with various markers such as enzymes, dyes, heavy metals, radioactive isotopes, etc. Avidin has four binding sites for biotin, and many biotin molecules can be incorporated on a given protein. This amplification principle can be useful to detect minute amounts (i.e., ng/mL or even pg/mL) of the marker disaccharides in the glycoproteins of rectal mucus obtained during digital rectal examination. Mucus glycoprotein containing the specific disaccharide will avidly bind to the lectins immobilized on a solid phase. A matrix formed by biotinylated lectins and enzyme-avidin D conjugate will bind to residual disaccharides on the immobilized glycoprotein lectins, while a suitable substrate will amplify the reaction.

Without further elaboration, it is believed that one skilled in the art can, using the preceding description, utilize the present invention to its fullest extent. The following preferred specific embodiments are, therefore, to be construed as merely illustrative, and not limitative of the remainder of the disclosure in any way whatsoever.

In the foregoing and in the following examples, all temperatures are set forth uncorrected in degrees Celsius and unless otherwise indicated, all parts and percentages are by weight.

In all of the tests described herein, sterile gloves should be worn; forceps, scissors, and all work surfaces should be scrupulously clean, membrane filters should be handled with forceps and filters must not be contaminated (even saliva may contaminate).

### EXAMPLES

**Example 1 -** A mucosal scrape sample is collected from a subject. The scrape sample is mixed with distilled or reverse osmosis water and applied to a test strip with galactose oxidase pre-embedded at a concentration of 100U/mL. The sample is allowed to remain on the test strip for 10 minutes after which point the test strip is rinsed with additional distilled water. In a separate container, a Schiff reagent is activated in solution after which the solution is added to the test strip. The sample is allowed to remain in contact with the Schiff reagent for 1 minute after which the test strip is rinsed with water and dried in open air or in an oven. Fig. 2 provides a depiction of the test sample processing. A color change (from white or colorless to magenta) is indicative of the presence of the carbohydrate marker.

**Example 2 -** A mucosal scrape sample is collected from a subject. The scrape sample is mixed with distilled or reverse osmosis water and added to a solution of galactose oxidase at a concentration of 100U/mL. The sample is allowed to remain in contact with the galactose oxidase for 10 minutes after which point the sample is added to a test strip with pre-embedded Schiff reagent. The test strip is rinsed with additional distilled water after which the Schiff reagent is activated and allowed to remain in contact with the sample for 1 minute after which the test strip is rinsed with water and dried in open air or in an oven. Fig. 3 provides a depiction of the test sample processing. A color change (from white or colorless to magenta) is indicative of the presence of the carbohydrate marker.

**Example 3** Following the procedure described above for the galactose oxidase strip test, the rectal mucus of 382 individuals either with known cancerous or precancerous conditions of the large intestine or other body site or who were not expressing any obvious signs or symptoms of a cancer or precancer were tested. The term "other body site" includes, but is not restricted to, the uterus, cervix, kidneys, head and neck, ovaries, breast, lymph nodes, stomach, testes, prostate, lungs, gallbladder, liver and pancreas.

**TABLE 1**

| summarizes the results of these tests. | | | | |
|---|---|---|---|---|
| Large Intestinal | | Other Body Site | High Risk | |
| Cancer | Polyp | Cancer | Symptomatic | Normal |
| 31/34 | 53/85 | 10/16 | 95/190 | 3/57 |
| 91.2% | 63.5% | 62.5% | 50% | 5.2% |

The data in the first line are the number of individuals tested positive/total number of individuals in each category. Note that only 5% of the apparently healthy individuals elicited a positive reaction.

In accordance with this invention, the mucus sample of each individual was obtained by digital rectal examination with the gloved finger lubricated with either normal saline or other common lubricants used for such procedure. The mucus on the examining finger was smeared on the protein-capturing membrane filter, reacted with galactose oxidase for 10 minutes at ambient temperature (25° C.), washed with deionized distilled water, reacted with basic fuchsin for 1 minute and then washed in tap water for 1 minute. The presence of a cancerous or precancerous condition is indicated by pink, magenta, or purple coloration of areas of the sample. Each sample which tested negative in this test by the absence of magenta coloration was then reacted with periodic acid for 5 minutes, washed with deionized distilled water and reacted again with basic fuchsin for 1 minute and washed with tap water. The positive reaction eliciting a pink to purple to magenta color is indicative of the fact that mucus glycoprotein had indeed been obtained but is negative with respect to the presence therein of a marker carbohydrate. The absence of pink, magenta, or purple coloration means sampling error and the individual is tested again with a fresh mucus sample.

In a separate independent study employing the above techniques, the presence of 9/11 stomach cancers, 4/4 cancers of the liver, gallbladder, common bile duct and pancreas were signaled by a positive test with the rectal mucus, galactose oxidase and basic fuchsin and, in the same manner, in another study, 12/18 patients with cancers of the stomach, pancreas and liver gave positive test results. In other studies, the rectal mucus of individuals with cancer of the ovary, breast or stomach tested positive for a marker protein.

Contemplated equivalents of the method of this invention is the use thereof as a primary screening test employing prostatic fluid, ejaculate, breast discharge, vaginal mucus, coughed up sputum, pancreatic and bile duct secretion for the detection of cancerous or precancerous conditions of the prostate, of the breast and of the cervix and/or ovaries, lungs, pancreas, liver and bile duct respectively, and the use of another galactose moiety specific oxidant instead of galactose oxidase.

**Example 4***-Hemagglutination Inhibition Test* -his standard hemagglutination inhibition test, e.g., as described in U.S. Pat. No. 4,857,457, can be used in the marker carbohydrate test employed in this invention.

In typical results with this test, no false negatives and far fewer false positives (about one-third vs. about 95) are obtained than in the conventional fecal occult blood test. A negative test result is checked for proper mucus sampling procedure by testing the sample for glycoprotein content according to this invention.

**Example 5-** *Latex Agglutination Test* -In this test, 500 µL of suspended latex beads (15.8 µ diameter, Sigma Chemical Co., St. Louis, Mo.) are centrifuged at 3,000 RPM for 15 seconds and the supernatant is decanted. 500 µg of lectin, e.g., PNA (Vector Laboratories Ltd., Burlingame, Calif.), is dissolved in 500 µL of carbonate buffer (pH 9.6) and added to the pellet of latex beads. The pellet is resuspended and incubated at 25° C. for 2 hours with occasional mild shaking to resuspend the beads and allow a more uniform binding. After incubation, the sample is centrifuged at 3,000 RPM for 15 seconds the supernatant decanted, and the pellet resuspended in PBS (pH 7.4). Any unbound PNA is washed off by repeating the previous step three times. The final pellet is suspended and diluted 1:10 in PBS.

For testing a mucus sample collected during digital rectal examination, 10 µL of mucus in PBS is added to an equal amount of the latex beads and placed on a glass slide. After five minutes of incubation at 25° C., the slide is read. An agglutination of the beads, indicating the presence of the marker disaccharide is read as positive for a cancerous condition, whereas no agglutination after 5 minutes indicates either the absence of the disaccharide and hence a cancer-free status or sampling error. The latter possibility is eliminated in accordance with this invention by assaying the mucus sample for glycoprotein content.

**Example 6** - *Biotinylated Lectin Avidin-Enzyme Assay* -Plant lectins, e.g., PNA, JAC, ACA, SNA, SRL, WGA, are dissolved in carbonate buffer (pH 9) to a final concentration of 100 ng/ml is used to cot the microtiter wells. 10 ng of lectins in 100 µL buffer are placed in each well and incubated at 37° C. for 2 hours. The wells are then washed off with phosphate buffered saline (PBS) pH 7.4, after which 100 µl of test mucus (dissolved in PBS) is added to the microtiter wells and the mixture is incubated at 37° C. for 1 hour. The wells are then washed three times with PBS to remove 100 µL of unbound mucus. Biotinylated lectins (1 µg/mL) is incubated for an additional hour at 37° C. in order to bind with residual marker carbohydrate (if any). The wells are washed three times with PBS to wash off unbound biotinylated lectins. Avidin-D-alkaline phosphatase (Vector Corporation, Burlingame, Calif.) is then added (100 µL/well, 1:50 dilution) to the wells and incubated for 1 hour at 37° C. Following 2 washed with PBS and 3 washes with bicarbonate buffer (pH 9.8), the substrate p-nitrophenyl phosphate (1 mg/mL) is added to the wells (100 µL/well). Optical absorbance at 405 nm is read after 30 minutes incubation at 37° C. Mucus from known cancer patients give position results while mucus from non-cancer patients give negative results.

In another example, samples are applied onto a nitrocellulose filter, and treated with 3% BSA-PBS for 1 hr. Thereafter, the filters were incubated with peroxidase-conjugated peanut lectin (PNA, Sigma, St. Louis, MO) at a concentration of 1 µg/ml in 0.1% BSA-PBS for 1 hr; 10 mL of PNA solution was used for each filter. The filters is washed thrice with 0.1% BSA-PBS (10 min each) and once with PBS alone. Chloronaphtol (Sigma) at 0.6 mg/ml PBS is then used as the substrate in the presence of 0.15% H₂O₂ and developed for 10 min at room temperature. Purple precipitate indicated positive staining. A computer-assisted image-analysis program is used to assess the staining for evaluation of the PNA positivity.

**Example 7 -** *Immunoassay using anti-Gal-GalNAc antibody* - The monoclonal antibodies against Gal-GalNAc (also known as TF antigen) are applied to Nunc 96-well plates. After washing in TBS containing 0.1% Tween 20, and blocking with this solution containing 2% bovine serum albumin, 100 uL of samples are added and incubated for 4 hours at room temperature. After washing in TBS containing 0.1% Tween 20, biotinylated asialofetuin (a standard source of TF) is added. Washed wells are incubated with avidin/peroxidase complex and peroxidase activity was measured by incubation in 2,2-azino-bis(3-ethylbenzthiazoline-6-sulfonic acid) liquid substrate system. The absorbance is read at 405 nm with an ELISA plate reader. The concentration of Gal-GalNAc (TF) is determined by interpolation of the absorbance values against a standard curve done with different dilutions of biotinylated asialofetuin.

The preceding examples can be repeated with similar success by substituting the generically or specifically described reactants and/or operating conditions of this invention for those used in the preceding samples.

### EQUIVALENTS

It is understood that the detailed examples and embodiments described herein are given by way of example for illustrative purposes only, and are in no way considered to be limiting to the disclosure. For example, the relative quantities of the ingredients may be varied to optimize the desired effects, additional ingredients may be added, and/or similar ingredients may be substituted for one or more of the ingredients described. Additional advantageous features and functionalities associated with the systems, methods, and processes of the present disclosure will be apparent from the appended claims.

## Claims

1. An in vitro screening method for rapidly testing a subject for a cancerous or precancerous condition, wherein the subject displays no obvious signs or symptoms of a cancerous or precancerous condition, the method comprising the steps of (a) assaying a portion of a biological sample obtained from an individual for the presence therein of glycoprotein containing at least one carbohydrate selected from the group consisting of beta-D-Gal-(1->3)-D-GalNAc, Fuc-alpha-1->2 Gal-Beta(1->4)-Fuc-alpha-1->3-GlcNAc, Fuc-Alpha-1->2Gal-beta-(1->4)-Fuc-alpha-1->3-GlcNAc-beta-(1->3)-Gal-beta-(1>4)-GlcNAc and Fuc-alpha-1->2-Gal-beta-(1->4)-Fuc-alpha-1->3-GlcNAc-beta-(1->3)-Gal-beta-(1->4)-Fuc-alpha-1->3-GlcNAc, by briefly subjecting the sample to oxidizing conditions which are capable of selectively oxidizing only the cyclic sugar moieties of any said marker carbohydrate present in the glycoprotein in the sample at a hydroxy group-bearing ring carbon atom thereof to form an aldehydic sugar moiety and then visualizing any aldehydic saccharide groups thus formed with a Schiff's base dye; (b) also assaying a portion of the biological sample for the presence therein of any glycoprotein by subjecting the sample to the oxidizing action of an oxidizing agent which oxidizes the saccharide moieties of any glycoprotein therein to aldehydic sugar moieties and then visualizing any thus-produced aldehydic sugar moieties, the presence of thereby formed aldehydic sugar moieties confirming the adequacy of the sampling and the absence therein of thereby formed aldehyde sugar moieties establishing that the negative test results were due to sampling error; (c) retesting the individual in the same manner with a fresh biological sample, if the first sample assays negative in steps (a) and (b); and (d) diagnosing the asymptomatic patient as having cancer or a precancerous condition based on visualizing a color change in the Schiff's base dye.

2. A method according to claim 1, wherein the assay comprises the steps of adsorbing the biological sample onto a protein-capturing water-insoluble substrate and then washing the substrate to remove non-immobilized components of the sample from the substrate, preferably wherein the insoluble substrate is a membrane filter.

3. A method according to claim 1 or 2, wherein the marker carbohydrate sugar moieties are assayed by selectively oxidizing the glycoprotein so as to selectively oxidize the primary hydroxy groups of any galactose moieties thereof to aldehydic groups and the thus-oxidized glycoprotein is then assayed for oxidized vicinal galactose moieties, preferably wherein the galactose moieties are oxidized with galactose oxidase, in particular wherein the oxidized vicinal galactose moieties are visualized with basic fuchsin.

4. A method according to anyone of the preceding claims, which is performed simultaneously on a plurality of biological samples obtained from a plurality of individuals as part of a field screening for cancer.

5. A method according to anyone of the preceding claims, wherein step (b) is conducted concurrently with step (a) on a different portion of the same biological sample; and wherein immediately thereafter, when a negative result is obtained in both of steps (a) and (b), immediately thereafter on another sample of biological sample obtained from the individual steps (a) and (b) are repeated, preferably wherein any aldehydic sugar moieties in a sample further oxidized with periodic acid are visualized with basic fuchsin.

6. A method according to anyone of the preceding claims, wherein prior to the assaying thereof the biological sample is adsorbed onto a protein-capturing water-insoluble substrate and the substrate is then washed to remove the non-immobilized components of the biological sample from the substrate; wherein the sample is assayed in step (a) by selectively oxidizing any glycoprotein in the sample so as to oxidize the primary hydroxy group of the galactose sugar moiety of any said carbohydrate therein to an aldehydic group and the thus-oxidized galactose moiety is then visualized with basic fuchsin; and wherein a sample which tests negative in that assay is further oxidized with periodic acid and the aldehydic sugar moiety in the thus-oxidized sample is visualized with basic fuchsin.

7. A method according to claim 6, wherein the oxidizing agent for the galactose moieties is present in the insoluble substrate or is applied directly thereto after the sample is applied thereto.

8. A method according to claim 7, wherein the insoluble substrate is a membrane filter, wherein the galactose sugar moieties are oxidized with galactose oxidase and wherein the galactose oxidase is applied directly to the membrane filter after the biological sample is applied thereto.

9. An in vitro method for screening for a cancerous or precancerous condition in an organ other than the large intestine of a human being, wherein the human being displays no obvious signs or symptoms of a cancerous or precancerous condition, the method comprising subjecting a sample of proteinaceous secretion or proteinaceous fluid other than rectal mucus associated with that organ, to an assay for the presence therein of a marker carbohydrate selected from the group consisting of beta-D-Gal-(1->3)-D-GalNAc, Fuc-alpha-1->2 Gal-Beta(1->4)-Fuc-alpha-1->3-GlcNAc, Fuc-Alpha-1->2 Gal-beta-(1->4)-Fuc-alpha-1->3-GlcNAc-beta-(1->3)-Gal-beta-(1>4)-GlcNAc and Fuc-alpha-1->2-Gal-beta-(1->4)-Fuc-alpha-1->3-GlcNAc-beta-(1->3)-Gal-beta-(1->4)-Fuc-alpha-1 - >3-GlcNAc.

10. A method according to claim 9, wherein prior thereto the proteinaceous secretion is adsorbed onto a protein-capturing water-insoluble substrate and the substrate is then washed to remove the nonimmobilized components of the sample from the substrate; wherein the sample is assayed for the marker carbohydrates by selectively oxidizing any glycoprotein in the sample with galactose oxidase so as to selectively oxidize the primary hydroxy group of any galactose sugar moiety of any said marker carbohydrate therein to an aldehydic group and the thus-oxidized galactose moiety is then visualized with basic fuchsin; and wherein a portion of the sample which tests negative in that assay is further oxidized with periodic acid and the aldehydic sugar moiety in the thus-oxidized sample is visualized with basic fuchsin.

11. A method according to claim 10, which comprises the portion of the sample of proteinaceous secretion which is oxidized with periodic acid is a different portion of the same sample which is oxidized with galactose oxidase and is oxidized concurrently therewith.

12. The method of anyone of the preceding claims, wherein the subject or human being is female, and the sample is vaginal or endocervical mucus.

13. The method of anyone of claims 1 to 11, wherein the human being is female, and the sample is nipple aspirate.

14. The method of anyone of claims 1 to 11 wherein the human being is male, and the sample is prostatic secretion or semen.

15. The method of anyone of claims 1 to 11, wherein the sample is selected from the group consisting of sputum,saliva, fluid bile duct, and pancreatic duct fluid aspirate.

## Patentansprüche

1. In-vitro-Screening-Verfahren zum schnellen Testen eines Subjekts auf einen kanzerösen oder präkanzerösen Zustand, wobei das Subjekt keine offensichtlichen Anzeichen oder Symptome eines kanzerösen oder präkanzerösen Zustands zeigt, wobei das Verfahren die Schritte umfasst: (a) Untersuchen eines Teils einer biologischen Probe, die von einem Individuum erhalten wurde, auf das Vorhandensein eines Glykoproteins darin, das mindestens ein Kohlenhydrat enthält, das aus der Gruppe ausgewählt ist, bestehend aus beta-D-Gal-(1->3)-D-GalNAc, Fuc-alpha-1->2 Gal-Beta(1->4)-Fuc-alpha-1->3-GlcNAc, Fuc-Alpha-1->2Gal-beta-(1->4)-Fuc-alpha-1->3-GlcNAc-beta-(1->3)-Gal-beta-(1>4)-GlcNAc und Fuc-alpha-1->2-Gal-beta-(1->4)-Fuc-alpha-1->3-GlcNAc-beta-(1->3)-Gal-beta-(1->4)-Fuc-alpha-1->3-GlcNAc, durch kurzes Unterwerfen der Probe oxidierenden Bedingungen, die in der Lage sind, selektiv nur die zyklischen Zuckergruppen irgendeines Markerkohlenhydrats, das in dem Glykoprotein in der Probe vorhanden ist, an einem hydroxygruppentragenden Ringkohlenstoffatom davon zu oxidieren, um eine aldehydische Zuckergruppe zu bilden, und anschließendes Sichtbarmachen irgendeiner so gebildeten aldehydischen Saccharidgruppe mit einem Schiff schen Basenfarbstoff; (b) auch Untersuchen eines Teils der biologischen Probe auf das Vorhandensein irgendeines Glykoproteins darin, indem die Probe der oxidierenden Wirkung eines Oxidationsmittels unterworfen wird, das die Saccharidgruppen irgendeines Glykoproteins darin zu Aldehydzuckergruppen oxidiert, und anschließendes Sichtbarmachen irgendwelcher auf diese Weise gebildeter Aldehydzuckergruppen, wobei das Vorhandensein irgendwelcher auf diese Weise gebildeter Aldehydzuckergruppen die Angemessenheit der Probeentnahme bestätigt und das Fehlen irgendwelcher auf diese Weise gebildeter Aldehydzuckergruppen darin feststellt, dass die negativen Testergebnisse auf einen Probenentnahmefehler zurückzuführen sind; (c) erneutes Testen des Individuums auf die gleiche Weise mit einer frischen biologischen Probe, wenn die erste Probe in den Schritten (a) und (b) negativ ausfällt; und (d) Diagnostizieren des asymptomatischen Patienten als an Krebs oder einem präkanzerösen Zustand leidend, basierend auf dem Sichtbarmachen einer Farbänderung des Schiff schen Basenfarbstoffs.

2. Verfahren nach Anspruch 1, wobei das Untersuchen die Schritte des Adsorbierens der biologischen Probe an ein Protein einfangendes, wasserunlösliches Substrat und anschließend das Waschen des Substrats umfasst, um nicht immobilisierte Komponenten der Probe von dem Substrat zu entfernen, wobei das unlösliche Substrat vorzugsweise ein Membranfilter ist.

3. Verfahren nach Anspruch 1 oder 2, wobei die Zuckerreste des Markerkohlenhydrats durch selektive Oxidation des Glykoproteins untersucht werden, um so die primären Hydroxygruppen irgendwelcher Galaktose-Reste davon selektiv zu aldehydischen Gruppen zu oxidieren, und das so oxidierte Glykoprotein anschließend auf oxidierte vicinale Galaktosereste untersucht wird, bevorzugt wobei die Galaktosereste mit Galaktoseoxidase oxidiert werden, insbesondere wobei die oxidierten vicinalen Galaktosereste mit basischem Fuchsin sichtbar gemacht werden.

4. Verfahren nach irgendeinem der vorhergehenden Ansprüche, das gleichzeitig an einer Vielzahl von biologischen Proben durchgeführt wird, die von einer Vielzahl von Individuen als Teil eines Feldscreenings auf Krebs erhalten werden.

5. Verfahren nach irgendeinem der vorhergehenden Ansprüche, wobei Schritt (b) gleichzeitig mit Schritt (a) an einem anderen Teil derselben biologischen Probe durchgeführt wird; und wobei unmittelbar danach, wenn in beiden Schritten (a) und (b) ein negatives Ergebnis erhalten wird, unmittelbar danach an einer anderen Probe einer biologischen Probe, die aus den einzelnen Schritten (a) und (b) erhalten wurde, wiederholt wird, bevorzugt wobei irgendwelche aldehydischen Zuckerreste in einer Probe, die weiter mit Perjodsäure oxidiert wurde, mit basischem Fuchsin sichtbar gemacht werden.

6. Verfahren nach irgendeinem der vorhergehenden Ansprüche, wobei die biologische Probe vor ihrer Untersuchung an ein proteinbindendes, wasserunlösliches Substrat adsorbiert wird und das Substrat anschließend gewaschen wird, um die nicht immobilisierten Bestandteile der biologischen Probe vom Substrat zu entfernen; wobei die Probe in Schritt (a) durch selektives Oxidieren irgendeines Glykoproteins in der Probe untersucht wird, um die primäre Hydroxygruppe des Galaktose-Zuckerrestes irgendeines der Kohlenhydrate darin zu einer aldehydischen Gruppe zu oxidieren, und der so oxidierte Galaktoserest anschließend mit basischem Fuchsin sichtbar gemacht wird; und wobei eine Probe, die in diesem Test negativ ist, weiter mit Perjodsäure oxidiert wird und der aldehydische Zuckerrest in der so oxidierten Probe mit basischem Fuchsin sichtbar gemacht wird.

7. Verfahren nach Anspruch 6, wobei das Oxidationsmittel für die Galaktosereste in dem unlöslichen Substrat vorhanden ist oder direkt darauf aufgetragen wird, nachdem die Probe darauf aufgetragen wurde.

8. Verfahren nach Anspruch 7, wobei das unlösliche Substrat ein Membranfilter ist, wobei die Galaktose-Zuckerreste mit Galaktoseoxidase oxidiert werden und wobei die Galaktoseoxidase direkt auf den Membranfilter aufgebracht wird, nachdem die biologische Probe darauf aufgebracht wurde.

9. In vitro-Verfahren zum Screening auf einen kanzerösen oder präkanzerösen Zustand in einem anderen Organ als dem Dickdarm eines Menschen, wobei der Mensch keine offensichtlichen Anzeichen oder Symptome eines kanzerösen oder präkanzerösen Zustands zeigt, wobei das Verfahren das Unterwerfen einer Probe einer proteinartigen Sekretion oder einer proteinartigen Flüssigkeit, die nicht Rektalschleim ist und mit diesem Organ assoziiert ist, einem Test auf das Vorhandensein eines Markerkohlenhydrats darin umfasst, das ausgewählt ist aus der Gruppe bestehend aus beta-D-Gal-(1->3)-D-GalNAc, Fuc-alpha-1->2 Gal-Beta(1->4)-Fuc-alpha-1->3-GlcNAc, Fuc-Alpha-1->2 Gal-beta-(1->4)-Fuc-alpha-1->3-GlcNAc-beta-(1->3)-Gal-beta-(1>4)-GlcNAc und Fuc-alpha-1->2-Gal-beta-(1->4)-Fuc-alpha-1->3-GlcNAc-beta-(1->3)-Gal-beta-(1->4)-Fuc-alpha-1->3-GlcNAc.

10. Verfahren nach Anspruch 9, wobei zuvor das proteinhaltige Sekret an ein proteinbindendes, wasserunlösliches Substrat adsorbiert wird und das Substrat anschließend gewaschen wird, um die nicht immobilisierten Komponenten der Probe vom Substrat zu entfernen; wobei die Probe auf die Markerkohlenhydrate untersucht wird, indem irgendein Glykoprotein in der Probe selektiv mit Galaktoseoxidase oxidiert wird, um die primäre Hydroxygruppe irgendeines Galaktosezuckerrestes irgendeines Markerkohlenhydrats darin selektiv zu einer aldehydischen Gruppe zu oxidieren, und der so oxidierte Galaktoserest anschließend mit basischem Fuchsin sichtbar gemacht wird; und wobei ein Teil der Probe, der in diesem Test negativ ist, mit Perjodsäure weiter oxidiert wird und der aldehydische Zuckerrest in der so oxidierten Probe mit basischem Fuchsin sichtbar gemacht wird.

11. Verfahren nach Anspruch 10, welches umfasst, dass der Teil der Probe der proteinhaltigen Sekretion, der mit Perjodsäure oxidiert wird, ein anderer Teil derselben Probe ist, der mit Galaktoseoxidase oxidiert wird und gleichzeitig damit oxidiert wird.

12. Verfahren nach einem der vorhergehenden Ansprüche, wobei das Subjekt oder der Mensch weiblich ist und die Probe Vaginal- oder Endozervikalschleim ist.

13. Verfahren nach einem der Ansprüche 1 bis 11, wobei der Mensch weiblich ist und die Probe ein Brustwarzenaspirat ist.

14. Verfahren nach einem der Ansprüche 1 bis 11, wobei der Mensch männlich ist und die Probe Prostatasekret oder Sperma ist.

15. Verfahren nach irgendeinem der Ansprüche 1 bis 11, wobei die Probe ausgewählt ist aus der Gruppe bestehend aus Sputum, Speichel, Gallenflüssigkeit und Pankreasgangflüssigkeitsaspirat.

## Revendications

1. Un procédé de dépistage in vitro pour tester rapidement un sujet pour une affection cancéreuse ou précancéreuse, dans lequel le sujet ne présente aucun signe ou symptôme évident d'une affection cancéreuse ou précancéreuse, le procédé comprenant les étapes consistant à (a) doser une portion d'un échantillon biologique obtenu à partir d'un individu pour la présence à l'intérieur de celui-ci d'une glycoprotéine contenant au moins un hydrate de carbone sélectionné dans le groupe constitué de bêta-D-Gal-(1->3)-D-GalNAc, Fuc-alpha-1->2 Gal-bêta(1->4)-Fuc-alpha-1->3-GlcNAc, Fuc-alpha-1->2 Gal-bêta-(1->4)-Fuc-alpha-1->3-GlcNAc-bêta-(1->3)-Gal-bêta-(1->4)-GlcNAc et Fuc-alpha-1->2-Gal-bêta-(1->4)-Fuc-alpha-1->3-GlcNAc-bêta-(1->3)-Gal-bêta-(1->4)-Fuc-alpha-1->3-GlcNAc, en soumettant brièvement l'échantillon à des conditions d'oxydation qui sont capables d'oxyder sélectivement uniquement les groupements de sucre cycliques de n'importe quel dit hydrate de carbone marqueur présent dans la glycoprotéine dans l'échantillon au niveau d'un atome de carbone de cycle porteur de groupe hydroxyle de celle-ci pour former un fragment de sucre aldéhydique et ensuite visualiser n'importe quels groupes saccharidiques aldéhydiques ainsi formés avec un colorant de base de Schiff ; (b) doser également une portion de l'échantillon biologique pour la présence à l'intérieur de celui-ci d'une glycoprotéine quelconque en soumettant l'échantillon à l'action oxydante d'un agent oxydant qui oxyde les fragments saccharidiques d'une glycoprotéine quelconque à l'intérieur de celui-ci en fragments de sucre aldéhydiques et ensuite visualiser n'importe quels fragments de sucre aldéhydiques ainsi produits, la présence de fragments de sucre aldéhydiques ainsi formés confirmant l'adéquation de l'échantillonnage et l'absence à l'intérieur de celui-ci de fragments de sucre aldéhydiques ainsi formés établissant que les résultats de test négatifs étaient dus à une erreur d'échantillonnage ; (c) retester l'individu de la même manière avec un échantillon biologique frais, si le premier échantillon est dosé négatif dans les étapes (a) et (b) ; et (d) diagnostiquer le patient asymptomatique comme ayant un cancer ou une affection précancéreuse sur la base de la visualisation d'un changement de couleur dans le colorant de base de Schiff.

2. Un procédé selon la revendication 1, dans lequel le dosage comprend les étapes d'adsorber l'échantillon biologique sur un substrat insoluble dans l'eau capturant les protéines et ensuite de laver le substrat pour éliminer les composants non immobilisés de l'échantillon du substrat, de préférence dans lequel le substrat insoluble est un filtre à membrane.

3. Un procédé selon la revendication 1 ou 2, dans lequel les groupements glucide-sucre marqueurs sont dosés en oxydant sélectivement la glycoprotéine de manière à oxyder sélectivement les groupes hydroxyle primaires de tout groupement galactose de celle-ci en groupes aldéhydes et la glycoprotéine ainsi oxydée est ensuite dosée pour des groupements galactose vicinaux oxydés, de préférence dans lequel les groupements galactose sont oxydés avec une galactose oxydase, en particulier dans lequel les groupements galactose vicinaux oxydés sont visualisés avec une fuchsine basique.

4. Un procédé selon l'une quelconque des revendications précédentes, qui est effectué simultanément sur une pluralité d'échantillons biologiques obtenus à partir d'une pluralité d'individus dans le cadre d'un dépistage de terrain pour le cancer.

5. Un procédé selon l'une quelconque des revendications précédentes, dans lequel l'étape (b) est effectuée simultanément avec l'étape (a) sur une portion différente du même échantillon biologique ; et dans lequel immédiatement après, lorsqu'un résultat négatif est obtenu dans les deux étapes (a) et (b), immédiatement après sur un autre échantillon d'échantillon biologique obtenu à partir des étapes individuelles (a) et (b) sont répétés, dans lequel, de préférence, tout groupement sucre aldéhyde dans un échantillon oxydé davantage avec un acide périodique est visualisé avec une fuchsine basique.

6. Un procédé selon l'une quelconque des revendications précédentes, dans lequel avant le dosage de celui-ci, l'échantillon biologique est adsorbé sur un substrat insoluble dans l'eau capturant les protéines et le substrat est ensuite lavé pour éliminer les composants non immobilisés de l'échantillon biologique du substrat ; dans lequel l'échantillon est dosé dans l'étape (a) en oxydant sélectivement toute glycoprotéine dans l'échantillon de manière à oxyder le groupe hydroxyle primaire du groupement galactose-sucre de tout dit glucide dans celui-ci en un groupe aldéhyde et le groupement galactose ainsi oxydé est ensuite visualisé avec une fuchsine basique ; et dans lequel un échantillon qui teste négatif dans ce dosage est davantage oxydé avec un acide périodique et le groupement sucre aldéhyde dans l'échantillon ainsi oxydé est visualisé avec une fuchsine basique.

7. Un procédé selon la revendication 6, dans lequel l'agent oxydant pour les groupements galactose est présent dans le substrat insoluble ou est appliqué directement à celui-ci après que l'échantillon a été appliqué à celui-ci.

8. Un procédé selon la revendication 7, dans lequel le substrat insoluble est un filtre à membrane, dans lequel les groupements galactose-sucre sont oxydés avec une galactose oxydase et dans lequel la galactose oxydase est appliquée directement au filtre à membrane après que l'échantillon biologique a été appliqué à celui-ci.

9. Un procédé in vitro pour le dépistage d'un état cancéreux ou précancéreux dans un organe autre que le gros intestin d'un être humain, dans lequel l'être humain ne présente aucun signe ou symptôme évident d'un état cancéreux ou précancéreux, le procédé comprenant le fait de soumettre un échantillon de sécrétion protéique ou de fluide protéique autre que le mucus rectal associé à cet organe, à un dosage pour la présence dans celui-ci d'un glucide marqueur choisi dans le groupe constitué de bêta-D-Gal-(1->3)-D-GaINAc, Fuc-alpha-1->2 Gal-bêta(1->4)-Fuc-alpha-1->3-GlcNAc, Fuc-alpha-1->2 Gal-bêta(1->4)-Fuc-alpha-1->3-GlcNAc-bêta-(1->3)-Gal-bêta-(1->4)-GlcNAc et Fuc-alpha-1->2-Gal-bêta-(1->4)-Fuc-alpha-1->3-GlcNAc-bêta-(1->3)-Gal-bêta-(1->4)-Fuc-alpha-1 ->3-GlcNAc.

10. Un procédé selon la revendication 9, dans lequel avant celui-ci, la sécrétion protéique est adsorbée sur un substrat insoluble dans l'eau capturant les protéines et le substrat est ensuite lavé pour éliminer les composants non immobilisés de l'échantillon du substrat ; dans lequel l'échantillon est dosé pour les glucides marqueurs en oxydant sélectivement toute glycoprotéine dans l'échantillon avec une galactose oxydase de manière à oxyder sélectivement le groupe hydroxyle primaire de tout groupement galactose-sucre de tout dit glucide marqueur dans celui-ci en un groupe aldéhyde et le groupement galactose ainsi oxydé est ensuite visualisé avec une fuchsine basique ; et dans lequel une portion de l'échantillon qui teste négatif dans ce dosage est davantage oxydée avec un acide périodique et le groupement sucre aldéhyde dans l'échantillon ainsi oxydé est visualisé avec une fuchsine basique.

11. Un procédé selon la revendication 10, qui comprend la portion de l'échantillon de sécrétion protéique qui est oxydée avec un acide périodique est une portion différente du même échantillon qui est oxydée avec une galactose oxydase et est oxydée simultanément avec celle-ci.

12. Le procédé selon l'une quelconque des revendications précédentes, dans lequel le sujet ou l'être humain est une femme, et l'échantillon est un mucus vaginal ou endocervical.

13. Le procédé selon l'une quelconque des revendications 1 à 11, dans lequel l'être humain est une femme, et l'échantillon est un aspirat de mamelon.

14. Le procédé selon l'une quelconque des revendications 1 à 11, dans lequel l'être humain est un homme, et l'échantillon est une sécrétion prostatique ou un sperme.

15. Le procédé selon l'une quelconque des revendications 1 à 11, dans lequel l'échantillon est choisi dans le groupe constitué par une expectoration, une salive, un conduit biliaire fluide, et un aspirat de fluide de conduit pancréatique.
